# EUROPEAN PATENT SPECIFICATION

(11) **EP 1 618 107 B1**
(45) Date of publication and mention of the grant of the patent: **30.06.2010**
(21) Application number: 04726587.1
(22) Date of filing: 08.04.2004
(51) Int. Cl.: C07D 471/04, C07D 403/14, A61K 31/4375, A61K 31/519

(54) **CONDENSED N-HETEROCYCLIC COMPOUNDS AND THEIR USE AS CRF RECEPTOR ANTAGONISTS**
KONDENSIERTE N-HETEROZYCLISCHE VERBINDUNGEN UND IHRE VERWENDUNG ALS CRF-REZEPTOR ANTAGONISTEN.
COMPOSES N-HETEROCYCLIQUES CONDENSES ET LEUR UTILISATION COMME ANTAGONISTES DU RECEPTEUR CRF

(30) Priority: 09.04.2003 GB 0308208; 07.07.2003 US 485322 P
(43) Date of publication of application: 25.01.2006
(73) Proprietor: SmithKline Beecham (Cork) Limited, Carrigaline Cork (IE); NEUROCRINE BIOSCIENCES, INC., San Diego, CA 92121-1102 (US)
(72) Inventor: ST-DENIS, Yves, I-37100 Verona (IT)
(74) Representative: Dolton, Peter Irving Ernest
(86) International application number: PCT/IB2004/001283
(87) International publication number: WO 2004/094419

(56) References cited:
- EP-A- 1 103 553
- WO-A-02/02549
- WO-A-98/05661
- WO-A-98/08846
- US-A1- 2002 049 207

## Description

The present invention relates to bicyclic derivatives, to processes for their preparation, to pharmaceutical compositions containing them and to their use in therapy.

The first corticotropin-releasing factor (CRF) was isolated from ovine hypothalami and identified as a 41-amino acid peptide (Vale et al., Science 213: 1394-1397,1981).

CRF has been found to produce profound alterations in endocrine, nervous and immune system function. CRF is believed to be the major physiological regulator of the basal and stress-release of adrenocorticotropic hormone ("ACTH"), Bendorphin and other proopiomelanocortin ("POMC")-derived peptides from the anterior pituitary (Vale et al., Science 213: 1394-1397,1981).

In addition to its role in stimulating the production of ACTH and POMC, CRF appears to be one of the pivotal central nervous system neurotransmitters and plays a crucial role in integrating the body's overall response to stress.

Administration of CRF directly to the brain elicits behavioral, physiological and endocrine responses identical to those observed for an animal exposed to a stressful environment.

Accordingly, clinical data suggests that CRF receptor antagonists may represent novel antidepressant and/or anxiolytic drugs that may be useful in the treatment of the neuropsychiatric disorders manifesting hypersecretion of CRF.

The first CRF receptor antagonists were peptides (see, e.g., Rivier et al., U.S. Patent No. 4,605,642; Rivier et al., Science 224: 889,1984). While these peptides established that CRF receptor antagonists can attenuate the pharmacological responses to CRF, peptide CRF receptor antagonists suffer from the usual drawbacks of peptide therapeutics including lack of stability and limited oral activity. More recently, small molecule CRF receptor antagonists have been reported.

WO 98/08846 describes compounds of general formula (C) having CRF antagonistic activity, 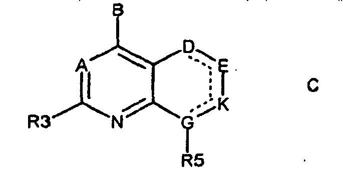 wherein A may be carbon, G may be nitrogen or carbon, B may be an amino derivative and the other groups have the meanings as defined.

Recently a patent application has been published as WO 02/08895 in which the following compounds, CRF antagonists, are objects of the Patent Application:

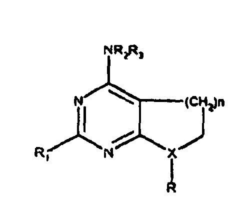

In particular, R₂ and R₃ with N may form a saturated or unsaturated heterocycle, which may be substituted by a 5-6 membered heterocycle, which may be substituted by 1 to 3 groups selected among: C1-C8 alkyl, halo C1-C2 alkyl, C1-C6 alkoxy, halogen, nitro or cyano,

Another recent patent application has been published as WO 03/008412 in which the following compounds, CRF antagonists, are objects of the Patent Application:

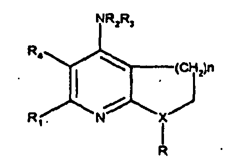

In particular, R₂ and R₃ with N may form a 5-14 membered heterocycle, which may be substituted by a 5-6 membered heterocycle, which may be saturated or may contain one to three double bonds, and which may be substituted by 1 or more groups such as C3-C7 cycloalkyl, C1-C6 alkyl, C1-C6 alkoxy, halo C1-C6 alkyl, C2-C6 alkenyl, C2-C6 alkynyl, halo C1-C6 alkoxy, hydroxy, halogen, nitro, cyano, or C(O)NR₆R₇.

None of the above references disclosed compounds falling into the scope of the present invention.

Due to the physiological significance of CRF, the development of biologically-active small molecules having significant CRF receptor binding activity and which are capable of antagonizing the CRF receptor remains a desirable goal. Such CRF receptor antagonists would be useful in the treatment of endocrine, psychiatric and neurologic conditions or illnesses, including stress-related disorders in general.

While significant strides have been made toward achieving CRF regulation through administration of CRF receptor antagonists, there remains a need in the art for effective small molecule CRF receptor antagonists. There is also a need for pharmaceutical compositions containing such CRF receptor antagonists, as well as methods relating to the use thereof to treat, for example, stress-related disorders. The present invention fulfills these needs, and provides other related advantages.

In particular the invention relates to novel compounds which are potent and specific antagonists of corticatropin-releaslng factor (CRF) receptors.

The present invention provides compounds of formula (1a) including stereoisomers and pharmaceutically acceptable salts or solvates thereof 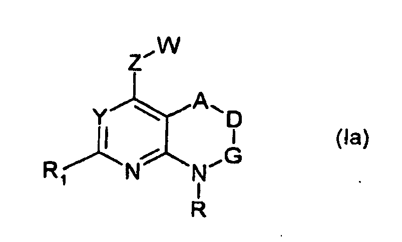 wherein
- R: is an aryl group selected from: 2,4-dichlorophenyl, 2-chloro-4-methylphenyl, 2-chloro-4- trifluoromethylphenyl, 2-chloro-4-methoxyphenyl, 2,4,5-trimethylphenyl, 2;4-dimethylphenyl, 2-methyl-4-methoxyphenyl, 2-methyl-4-ethoxyphenyl, 2-methyl-4-isopropoxyphenyl, 2- methyl-4-hydroxyphenyl, 2-methyl-4-chlorophenyl, 2-methyl-4-trifluoromethylphenyl, 2,4- dimethoxyphenyl, 2-methoxy-4-trifluoromethylphenyl, 2-methoxy-4-chlorophenyl, 3-methoxy- 4-chlorophenyl, 2,5-dimethoxy-4-chlorophenyl, 2-methoxy-4-isopropylphenyl, 2-methoxy-4- trifluoromethylphenyl, 2-methoxy-4-isopropylphenyl, 2-methoxy-4-methylphenyl, 2- trifluoromethyl-4-chlorophenyl, 2,4-bis-trifluoromethylphenyl, 2-trifluoromethyl-4- methylphenyl, 2-trifluoromethyl-4-methoxyphenyl, 2-difluoromethyl-4-methoxyphenyl, 2- bromo-4-isopropylphenyl, 2-methyl-4-cyanophenyl, 2-chloro-4-cyanophenyl, 2- trifluoromethyl-4-cyanophenyl, 2-trifluoromethoxy-4-cyanophenyl, 2-ethyl-4-cyanophenyl, 2- methyl-4-trifluoromethoxyphenyl, 4-methyl-6-dimethylaminopyridin-3-yl, 2,6-bismethoxy- pyridin-3-yl, 2-methyl-6-methoxy-pyridin-3-yl, 2-trifluoromethyl-6-methoxy-pyridin-3-yl 3- chloro-5-trichloromethyl-pyridin-2-yl, 2-methyl-4-(pyrazol-1-yl)-phenyl, 2-methoxy-4-(pyrazol- 1-yl)-phenyl, 2,4,6-trimethoxyphenyl, 2-methyl-4,5-benzodioxolyl, 2-methyl-3,4-benzodioxolyl;
- R₁: is -CH₃;
- R₃: is hydrogen or C1-C6 alkyl;
- R₄: is hydrogen or C1-C6 alkyl;
- R₅: is a C1-C6 alkyl, halo C1-C6 alkyl, C1-C6 alkoxy, halo C1-C6 alkoxy, C3-C7 cycloalkyl, hydroxy, halogen., nitro, cyano, -NR₃R₄:
- R₇: is hydrogen;
- R₈: is hydrogen;
- R₉: is hydrogen;
- R₁₀: is hydrogen;
- R₁₁: is hydrogen;
- R₁₂: is hydrogen;
- R₁₃: is hydrogen;
- R₁₄: is R₃;
- D: is CR₈R₉;
- G: is CR₁₀R₁₁ or is CR₁₀;
- A: is CR₁₂R₁₃;
- X: is carbon or nitrogen;
- Y: is nitrogen or -CR₇;
- W: is 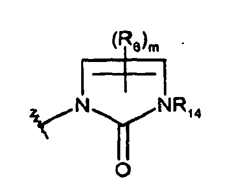
- Z: is pyrazole which may be substituted by 1 to 2 R₅ groups;
- m: is 0.

The compounds of the present invention may be in the form of and/or may be administered as a pharmaceutically acceptable salt. For a review on suitable salts see Berge et al, J. Pharm. Sci., 1977, 66, 1-19.

Typically, a pharmaceutical acceptable salt may be readily prepared by using a desired acid or base as appropriate. The salt may precipitate from solution and be collected by filtration or may be recovered by evaporation of the solvent.

Suitable addition salts are formed from acids which form non-toxic salts and examples are hydrochloride, hydrobromide, hydroiodide, sulphate, bisulphate, nitrate, phosphate, hydrogen phosphate, acetate, maleate, malate, fumarate, lactate, tartrate, citrate, formate, gluconate, succinate, piruvate, oxalate, oxaloacetate, trifluoroacetate, saccharate, benzoate, methansulphonate, ethanesulphonate, benzenesulphonate, p-toluensulphonate, methanesulphonic, ethanesulphonic, p-toluenesulphonic, and isethionate.

Pharmaceutically acceptable base salts include ammonium salts, alkali metal salts such as those of sodium and potassium, alkaline earth metal salts such as those of calcium and magnesium and salts with organic bases, including salts of primary, secondary and tertiary amines, such as isopropylamine, diethylamine, ethanolamine, trimethylamine, dicyclohexyl amine and N-methyl-D-glucamine.

Those skilled in the art of organic chemistry will appreciate that many organic compounds can form complexes with solvents in which they are reacted or from which they are precipitated or crystallized. These complexes are known as "solvates". For example, a complex with water is known as a "hydrate". Solvates of the compound of the invention are within the scope of the invention.

With regard to stereoisomers, the compounds of structure (I) may have one or more asymmetric carbon atom and may occur as recemates, racemic mixtures and as individual enantiomers or diastereomers. All such isomeric forms are included within the present invention, including mixtures thereof.

Where a compound of the invention contains an alkenyl or alkenylene group, cis (E) and trans (Z) isomerism may also occur. The present invention includes the indivisual stereoisomers of the compound of the invention and, where appropriate, the individual tautomeric forms thereof, together with mixtures thereof.

Separation of diastereoisomers or cis and trans isomers may be achieved by conventional techniques, e.g. by fractional crystallisation, chromatography or H.P.L.C. of a stereoisomeric mixture of the agent may also be prepared from a corresponding optically pure intermediate or by resolution, such as H.P.L.C. of the corresponding racemate using a suitable chiral support or by fractional crystallisation of the diastereoisomeric salts formed by reaction of the corresponding racemate with a suitable optically active acid or base, as appropriate.

Those skilled in the art of organic chemistry will appreciate that many organic compounds can form complexes with solvents in which they are reacted or from which they are precipitated or crystallized. These complexes are known as "solvates". For example, a complex with water is known as a "hydrate". Solvates of the compounds of the invention are within the scope of the invention.

Furthermore, some of the crystalline forms of the compounds of structure (I) may exist as polymorphs, which are included in the present invention.

The term C1-C6 alkyl as used herein as a group or a part of the group refers to a linear or branched alkyl group containing from 1 to 6 carbon atoms; examples of such groups include methyl, ethyl, propyl, lsopropyl, n-butyl, isobutyl, tert butyl, pentyl or hexyl.

The term C3-C7 cycloalkyl group means a non aromatic monocyclic hydrocarbon ring of 3 to 7 carbon atom such as, for example, cyclopropyl, cyclobutyl, cyclopentyl, cyclohexyl or cycloheptyl; while unsaturated cycloalkyls include cyclopentenyl and cyclohexenyl, and the like.

The term halogen refers to a fluorine, chlorine, bromine or iodine atom.

The term halo C1-C6 alkyl, or halo C1-C2 alkyl means an alkyl group having one or more carbon atoms and wherein at least one hydrogen atom is replaced with halogen such as for example a trifluoromethyl group and the like.

The term C1-C6 alkoxy group may be a linear or a branched chain alkoxy group, for example methoxy, ethoxy, propoxy, prop-2-oxy, butoxy, but-2-oxy or methylprop-2-oxy and the like.

The term halo C1-C6 alkoxy group may be a C1-C6 alkoxy group as defined before substituted with at least one halogen, preferably fluorine, such as OCHF₂, or OCF₃,

Preferred compounds according to the invention are:
1-{1-[8-(2,4-dichlorophenyl)-2-methyl-5,6,7,8-tetrahydropyrido[2,3-*d*]pyrimidin-4-yl]-1*H*-pyrazol-3-yl}-2-imidazolidinone;
1-{1-[8-(2,4-dichlorophenyl)-2-methyl-5,6,7,8-tetrahydro-4-quinazolinyl]-1*H*-pyrazol-3-yl}-2-imidazolidinone;
1-{1-[8-(2,4-dichlorophenyl)-2-methyl-5,6,7,8-tetrahydro-1,8-naphthyridin-4-yl}-1*H*-pyrazol-3-yl}-2-imidazolidinone.

In general, the compounds of structure (I) may be made according to the organic synthesis techniques known to those skilled in this field, as well as by the representative methods set forth in the Examples.

Compounds of formula (I), and salts and solvates thereof, may be prepared by the general methods outlined hereinafter. In the following description, the groups R, R₁, R₂, R₃. R₄, R₅, R₆, R₇, R₈, R₉, R₁₀, R₁₁, A, D, G, X, Y. W, Z and m have the meanings as previously defined for compounds of formula (I), unless otherwise stated.

Compounds of formula (1a) in which Y=N, A, D, and G are -CH₂, may be prepared according to the steps as illustrated in the following Scheme 1. 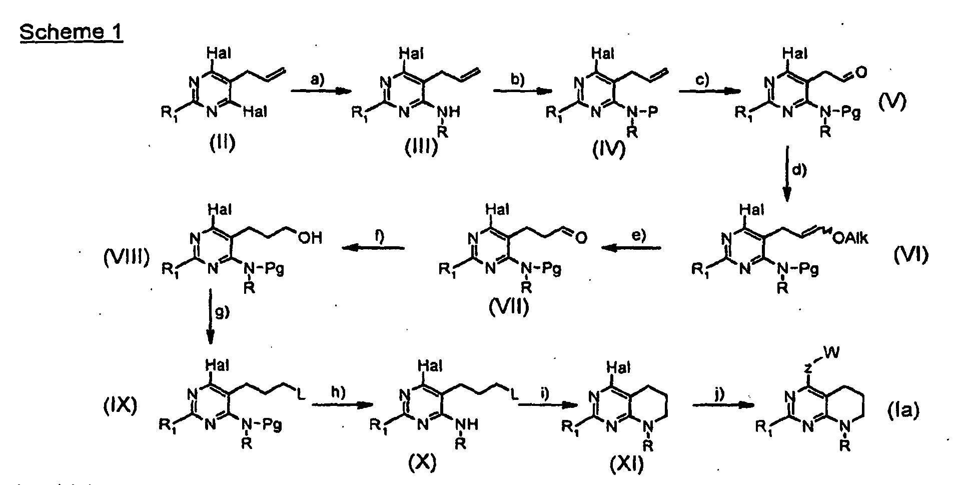 in which
- step a: stands for the nucleophilic substitution with a suitable amine (such as a substituted aniline) of compounds of formula (II), in basic conditions (such as sodium hydride in a polar aprotic solvent) to give compounds (III);
- step b: stands for the protection of the amino group with a suitable protecting group (such as a BOC group);
- step c: stands for the oxidation of the double bond with a suitable oxidizing agent (such as ozone in a polar protic solvent) to give the aldehyde of compounds (V);
- step d + e: stands for formation of the aldehyde group of compounds (VII) through formation of the enol ether by Wittig reaction in the usual conditions, followed by acid hydrolysis (step e);
- step f: stands for the reduction of the aldehyde group of compounds (VII) to the alcohol of compounds (VIII) with a suitable reducing agent (such as sodium borohydride);
- step g: stands for the conversion of the alcohol of compounds (VIII) into a suitable leaving group (such as, for example, a halogen or reactive residue of sulphonic acid (e.g. mesylate, tosylate), preferably mesylate);
- step h: stands for the deprotection of the amino group of compounds (IX);
- step i: stands for the intramolecular cyclization to give the cyclized compounds (X)
- step j: stands for conversion of the halogen derivative, preferably chloride, into compounds (1a), by reaction with the suitable reactive -Z-W derivative, in basic conditions (such as, for example, sodium hydride in a polar solvent).

Compounds of formula (Id) in which Y=N, A, D, and G are -CH₂, may be prepared according to the steps as illustrated in the following Scheme 2. 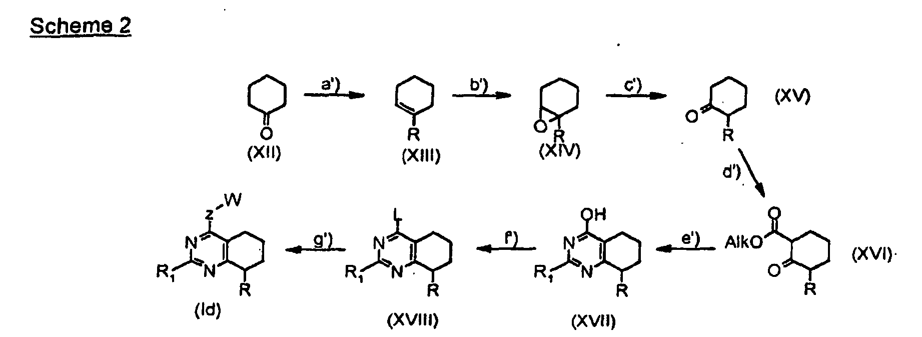 in which
- step a': stands for the conversion of the ketone of compounds (XII) into the enol ether (such as a triflate) followed by a metal catalyzed coupling reaction (such as a Suzuki reaction with an aryl boronic acid);
- step b': stands for the oxidation of the double bond of compound (XIII) with a suitable oxidizing agent (such as m-chloro-per-benzoic acid) to give the epoxide of compounds (XIV);
- step c': stands for the acid catalyzed rearrangement of epoxide of compounds (XIV) to give the ketone of compounds (XV);
- step d': stands for the acylation of compound (XV) to give an ester group (such as acylation with ethyl cyanoformate to give the ethyl ester derivative);
- step e': stands for the cyclisation of the β-ketoester of formula (XVI) with a salt (e.g: hydrochloride) of a substituted amidine (such as acetamidine hydrochloride) in order to form the pyrimidine compound (XVII);
- step f': stands for conversion of the hydroxy group into an halogen by the halogenation reaction carried out using, for example, treatment with PO(Hal)₃, wherein Hal is preferably chlorine;
- step g': stands for conversion of the leaving group L, selected in a group consisting from: halogen or reactive residue of sulphonic acid (e.g. mesylate, tosylate), preferably chloride, in the compounds (Id), by reaction with the suitable reactive -Z-W derivative, in basic conditions (such as, for example, sodium hydride in a polar solvent).

Compounds of formula (la) may be prepared according to the steps as illustrated in the following Scheme 3. 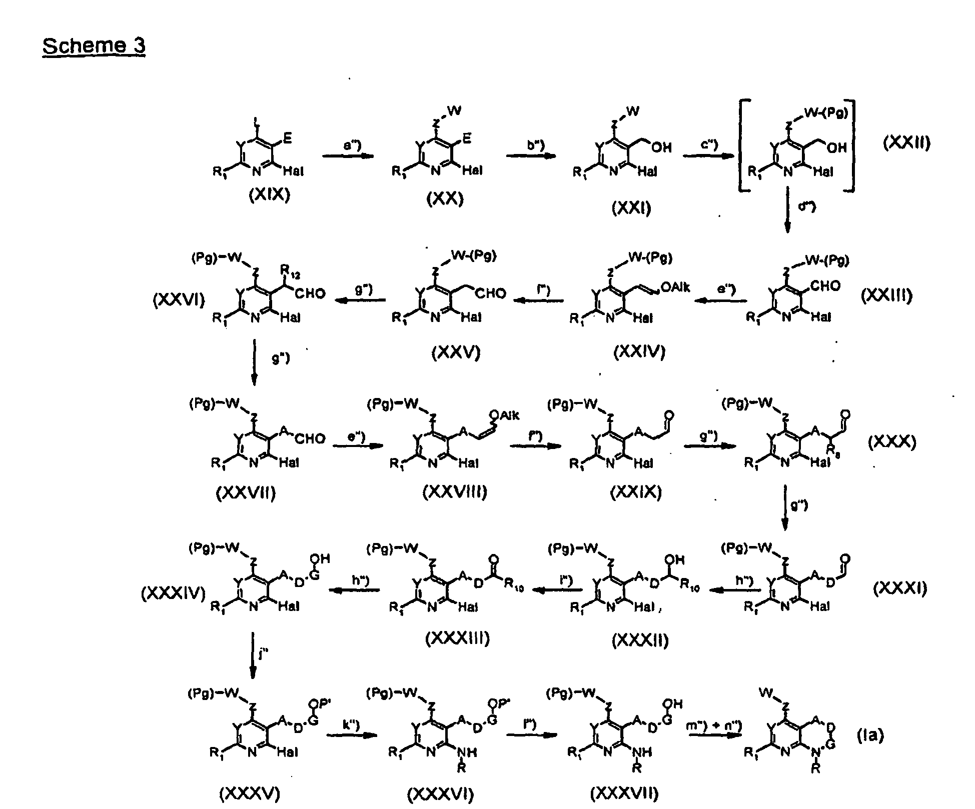 in which
- step a": stands for conversion of the leaving group L of compounds (XIX), selected in a group consisting from: halogen or reactive residue of sulphonic acid (e.g. mesylate, tosylate), preferably chloride, in the compounds (VIII), by reaction with the suitable Z-W derivative;
- step b": stands for reduction of the ester group (E) with a suitable reducing agent (such as DIBAI-H) to hydroxy group of compounds (XXI),
- step c": stands for suitable protection of an NH group eventually present in W group with a Pg group, such as a p-methoxybenzyl group;
- step d": stands for oxidation of the hydroxy group with a suitable oxidizing agent (such as Dess-Martin periodinane) to the aldehyde group of compounds (XXIII);
- steps e" + f": stands for formation of the aldehyde group of compounds (XXV) and (XXVII) by Wittig reaction in the usual conditions, through formation of enol ether followed by acid hydrolysis (step f);
- step g": stands for the optional alkylation of the α position of the aldehyde by deprotonation with a suitable base (such as LiN(SiMe₃)₂), followed by the addition of a suitable alkylating agent (such as Mel) to form the alkylated aldehyde of compounds (XXVI), (XXVII);
- step h": stands for the conversion of the aldehyde group by a Grignard reagent (such as MeMgBr) into an alcohol group of compounds (XXXII) and (XXXIV);
- step I": stands for oxidation of the hydroxy group with a suitable oxidizing agent (such as Dess-Martin periodinane) to the ketone group of compounds (XXXIII);
- step j": stands for conversion of the hydroxy group in the suitable protecting group of compounds (XXXV) (such as TBS: *tert*-butyldimethylsilyl);
- step k": stands for a Buchwald coupling reaction with the suitable amine RNH₂ to give the compounds of formula (XXXVI);
- step I": stands for the deprotection reaction to give the hydroxy group of compounds (XXXVII);
- step m": stands for intramolecular cyclisation after conversion of the hydroxy group of compounds (XXXVII) In a suitable leaving group (such as bromide, by reaction with CBr₄ and PPh₃) to give the final compounds (1a);
- step n": stands for the deprotection reaction of the protected NH group eventually present in W group, to give final compounds (la).

Compounds of formula (II), (XII) and (XIX) are known compounds or may be prepared according to known method in the literature.

Those skilled in the art will appreciate that in the preparation of the compound of the invention or a solvate thereof it may be necessary and/or desirable to protect one or more sensitive groups in the molecule to prevent undesirable side reactions. Suitable protecting groups for use according to the present invention are well known to those skilled in the art and may be used in a conventional manner. See, for example, "Protective groups in organic synthesis" by T.W. Greene and P.G.M. Wuts (John Wiley & sons 1991) or "Protecting Groups" by P.J. Kocienski (Georg Thieme Verlag 1994). Examples of suitable amino protecting groups include acyl type protecting groups (e.g. formyl, trifluoroacetyl, acetyl), aromatic urethane type protecting groups (e.g. benzyloxycarbonyl (Cbz) and substituted Cbz), aliphatic urethane protecting groups (e.g. 9-fluorenylmethoxycarbonyl (Fmoc), t-butyloxycarbonyl (Boc), isopropyloxycarbonyl, cyclohexyloxycarbonyl) and alkyl type protecting groups (e.g. benzyl, trityl, chlorotrityl). Examples of suitable oxygen protecting groups may include for example alky silyl groups, such as trimethylsilyl or tert-butyldimethylsilyl; alkyl ethers such as tetrahydropyranyl or tert-butyl; or esters such as acetate Pharmaceutical acceptable salts may also be prepared from other salts, including other pharmaceutically acceptable salts, of the compound of formula (I) using conventional methods.

The compounds of formula (I) may readily be isolated in association with solvent molecules by crystallisation or evaporation of an appropriate solvent to give the corresponding solvates.

When a specific enantiomer of a compound of general formula (I) is required, this may be obtained for example by resolution of a corresponding enantiomeric mixture of a compound of formula (I) using conventional methods. Thus the required enantiomer may be obtained from the racemic compound of formula (I) by use of chiral HPLC procedure.

The subject invention also includes isotopically-labelled compounds, which are identical to those recited in formula (I) and following, but for the fact that one or more atoms are replaced by an atom having an atomic mass or mass number different from the atomic mass or mass number usually found in nature. Examples of isotopes that can be incorporated into compounds of the invention and pharmaceutically acceptable salts thereof include isotopes of hydrogen, carbon, nitrogen, oxygen, phosphorous, sulphur, fluorine, iodine, and chlorine, such as ²H, ³H, ¹¹C, ¹³C, ¹⁴C, ¹⁵N, ¹⁷O, ¹⁸O, ³¹P, ³²P, ³⁵S, ¹⁸F, ³⁸Cl, ¹²³I and ¹²⁵I.

Compounds of the present invention and pharmaceutically acceptable salts of said compounds that contain the aforementioned isotopes and/or other isotopes of other atoms are within the scope of the present invention. Isotopically-labelled compounds of the present invention, for example those into which radioactive isotopes such as ³H, ¹⁴C are incorporated, are useful in drug and/or substrate tissue distribution assays. Tritiated, i.e., ³H, and carbon-14, i.e., ¹⁴C, isotopes are particularly preferred for their ease of preparation and detectability. ¹¹C and ¹⁸F isotopes are particularly useful in PET (positron emission tomography), and ¹²⁵I isotopes are particularly useful in SPECT (single photon emission computerized tomography), all useful in brain imaging. Further, substitution with heavier Isotopes such as deuterium, i.e., ²H, can afford certain therapeutic advantages resulting from greater metabolic stability, for example increased *in vivo* half-life or reduced dosage requirements and, hence, may be preferred in some circumstances. Isotopically labelled compounds of formula I and following of this invention can generally be prepared by carrying out the procedures disclosed in the Schemes and/or in the Examples below, by substituting a readily available isotopically labelled reagent for a non-isotopically labelled reagent.

The effectiveness of a compound as a CRF receptor antagonist may be determined by various assay methods. Suitable CRF antagonists of this invention are capable of inhibiting the specific binding of CRF to its receptor and antagonizing activities associated with CRF. A compound of structure (I) may be assessed for activity as a CRF antagonist by one or more generally accepted assays for this purpose, including (but not limited to) the assays disclosed by DeSouza et al. (J. Neuroscience 7: 88,1987) and Battaglia et al. (Synapse 1: 572,1987).

The CRF receptors-binding assay may be performed by using the homogeneous technique of scintillation proximity (SPA). The ligand binds to recombinant membrane preparation expressing the CRF receptors which in turn bind to wheatgerm agglutinin coated SPA beads. In the Experimental Part will be disclosed the details of the experiments.

With reference to CRF receptor binding affinities, CRF receptor antagonists of this invention have a Ki less than 10 µM.

Compounds of the invention are useful in the treatment of central nervous system disorders where CRF receptors are involved. In particular In the treatment or prevention of major depressive disorders including bipolar depression, unipolar depression, single or recurrent major depressive episodes with or without psychotic features, catatonic features, melancholic features, atypical features or postpartum onset, the treatment of anxiety and the treatment of panic disorders. Other mood disorders encompassed within the term major depressive disorders include dysthymic disorder with earthy or late onset and with or without atypical features, neurotic depression, post traumatic stress disorders, post operative stress and social phobia; dementia of the Alzheimer's type, with early or late onset, with depressed mood; vascular dementia with depressed mood; mood disorders induced by alcohol, amphetamines, cocaine, hallucinogens, inhalants, opioids, phencyclidine, sedatives, hypnotics, anxiolytics and other substances; schizoaffective disorder of the depressed type; and adjustment disorder with depressed mood. Major depressive disorders may also result from a general medical condition including, but not limited to, myocardial infarction, diabetes, miscarriage or abortion, etc.

Compounds of the invention are also useful in the treatment or prevention of schizophrenic disorders including paranoid schizophrenia, disorganised schizophrenia, catatonic schizophrenia, undifferentiated schizophrenia, residual schizoprenia.

Compounds of the invention are useful as analgesics. In particular they are useful in the treatment of traumatic pain such as postoperative pain; traumatic avulsion pain such as brachial plexus; chronic pain such as arthritic pain such as occurring in osteo-, rheumatoid or psoriatic arthritis; neuropathic pain such as post-herpetic neuralgia, trigeminal neuralgia, segmental or intercostal neuralgia, fibromyalgia, causalgia, peripheral neuropathy, diabetic neuropathy, chemotherapy-induced neuropathy, AIDS related neuropathy, occipital neuralgia, geniculate neuralgia, glossopharyngeal neuralgia, reflex sympathetic dystrophy, phantom limb pain; various forms of headache such as migraine, acute or chronic tension headache, temporomandibular pain, maxillary sinus pain, cluster headache; odontalgia; cancer pain; pain of visceral origin; gastrointestinal pain; nerve entrapment pain; sport's injury pain; dysmennorrhoea; menstrual pain; meningitis; arachnoiditis; musculoskeletal pain; low back pain e.g. spinal stenosis; prolapsed disc; sciatica; angina; ankylosing spondyolitis; gout; burns; scar pain; itch; and thalamic pain such as post stroke thalamic pain.

Compounds of the invention are also useful for the treatment of dysfunction of appetite and food intake and in circumstances such as anorexia, anorexia nervosa and bulimia.

Compounds of the invention are also useful in the treatment of sleep disorders including dysomnia, insomnia, sleep apnea, narcolepsy, and circadian rhythmic disorders.

Compounds of the invention are also useful in the treatment or prevention of cognitive disorders. Cognitive disorders include dementia, amnestic disorders and cognitive disorders not otherwise specified.

Furthermore compounds of the invention are also useful as memory and/or cognition enhancers in healthy humans with no cognitive and/or memory deficit.

Compounds of the invention are also useful in the treatment of tolerance to and dependence on a number of substances. For example, they are useful in the treatment of dependence on nicotine, alcohol, caffeine, phencyclidine (phencyclidine like compounds), or in the treatment of tolerance to and dependence on opiates (e.g. cannabis, heroin, morphine) or benzodiazepines; in the treatment of cocaine, sedative ipnotic, amphetamine or amphetamine- related drugs (e.g. dextroamphetamine, methylamphetamine) addiction or a combination thereof.

Compounds of the invention are also useful as anti-inflammatory agents. In particular they are useful in the treatment of inflammation in asthma, influenza, chronic bronchitis and rheumatoid arthritis; in the treatment of inflammatory diseases of the gastrointestinal tract such as Crohn's disease, ulcerative colitis, postoperative gastric ileus (POI), inflammatory bowel disease (IBD) and non-steroidal anti-inflammatory drug induced damage; inflammatory diseases of the skin such as herpes and eczema; inflammatory diseases of the bladder such as cystitis and urge incontinence; and eye and dental inflammation.

Compounds of the invention are also useful in the treatment of allergic disorders, in particular allergic disorders of the skin such as urticaria, and allergic disorders of the airways such as rhinitis.

Compounds of the invention are also useful in the treatment of emesis, i.e. nausea, retching and vomiting. Emesis includes acute emesis, delayed emesis and anticipatory emesis. The compounds of the invention are useful in the treatment of emesis however induced. For example, emesis may be induced by drugs such as cancer chemotherapeutic agents such as alkylating agents, e.g. cyclophosphamide, carmustine, lomustine and chlorambucil; cytotoxic antibiotics, e.g. dactinomycin, doxorubicin, mitomycin-C and bleomycin; anti-metabolites, e.g. cytarabine, methotrexate and 5- fluorouracil; vinca alkaloids, e.g. etoposide, vinblastine and vincristine; and others such as cisplatin, dacarbazine, procarbazine and hydroxyurea; and combinations thereof; radiation sickness; radiation therapy, e.g. irradiation of the thorax or abdomen, such as in the treatment of cancer, poisons; toxins such as toxins caused by metabolic disorders or by Infection, e.g. gastritis, or released during bacterial or viral gastrointestinal infection; pregnancy; vestibular disorders, such as motion sickness, vertigo, dizziness and Meniere's disease; post-operative sickness; gastrointestinal obstruction; reduced gastrointestinal motility; visceral pain, e.g. myocardial infarction or peritonitis; migraine; increased intercranial pressure; decreased intercranial pressure (e.g. altitude sickness); opioid analgesics, such as morphine; and gastro-oesophageal reflux disease, acid indigestion, over-indulgence of food or drink, acid stomach, sour stomach, waterbrash/regurgitation, heartburn, such as episodic heartburn, nocturnal heartburn, and meal-induced heartburn and dyspepsia.

Compounds of the invention are of particular use in the treatment of gastrointestinal disorders such as irritable bowel syndrome (IBS); skin disorders such as psoriasis, pruritis and sunburn; vasospastic diseases such as angina, vascular headache and Reynaud's disease; cerebral ischeamia such as cerebral vasospasm following subarachnoid haemorrhage; fibrosing and collagen diseases such as scleroderma and eosinophilic fascioliasis; disorders related to immune enhancement or suppression such as systemic lupus erythematosus and rheumatic diseases such as fibrositis; and cough.

Compounds of the invention are useful for the treatment of neurotoxic injury which follows cerebral stroke, thromboembolic stroke, hemorrhagic stroke, cerebral ischemia, cerebral vasospam, hypoglycemia, hypoxia, anoxia, perinatal asphyxia cardiac arrest.

The invention therefore provides a compound of formula (I) or a pharmaceutically acceptable salt or solvate thereof for use in therapy, in particular in human medicine.

There is also provided as a further aspect of the invention the use of a compound of formula (I) or a pharmaceutically acceptable salt or solvate thereof in the preparation of a medicament for use in the treatment of conditions mediated by CRF.

In an alternative or further aspect there is provided a methods for the treatment of a mammal, including man, in particular in the treatment of condition mediated by CRF, comprising administration of an effective amount of a compound of formula (I) or a pharmaceutically acceptable salt or a solvate thereof.

While it is possible that, for use in therapy, a compound of the present invention may be administered as the raw chemical, it is preferable to present the active ingredient as a pharmaceutical formulation e. g. when the agent is in admixture with a suitable pharmaceutical excipient, diluent or carrier selected with regard to the intended route of administration and standard pharmaceutical practice.

In a further aspect, the invention provides a pharmaceutical composition comprising at least one compound of the invention or a pharmaceutically acceptable derivative thereof in association with a pharmaceutically acceptable carrier and/or excipient. The carrier and/or excipient must be "acceptable" in the sense of being compatible with the other ingredients of the formulation and not deletrious to the receipient thereof.

Accordingly, the present invention further provides a pharmaceutical formulation comprising at least one compound of the invention or a pharmaceutically acceptable derivative thereof, in association with a pharmaceutically acceptable carrier and/or excipient. The carrier and/or excipient must be "acceptable" in the sense of being compatible with the other ingredients of the formulation and not deletrious to the receipient thereof.

There is further provided by the present invention a process of preparing a pharmaceutical composition, which process comprises mixing at least one compound of the invention or a pharmaceutically acceptable derivative thereof, together with a pharmaceutically acceptable carrier and/or excipient.

The pharmaceutical compositions may be for human or animal usage in human and veterinary medicine and will typically comprise any one or more of a pharmaceutically acceptable diluent, carrier or excipient. Acceptable carriers or diluents for therapetic use are well known in the pharmaceutical art, and are described, for example, in Remington's Pharmaceutical Sciences, Mack Publishing Co. (A. R. Gennaro edit. 1985). The choice of pharmaceutical carrier, excipient or diluent can be selected with regard to the intended route of administration and standard pharmaceutical practice. The pharmaceutical compositions may comprise as - or in addition to - the carrier, excipient or diluent any suitable binder(s), lubricant(s), suspending agent(s), coating agent(s), solubilising agent(s).

Preservatives, stabilisers, dyes and even flavouring agents may be provided in the pharmaceutical composition. Examples of preservatives include sodium benzoate, sorbic acid and esters of p-hydroxybenzoic acid. Antioxidants and suspending agents may be also used. There may be different composition/formulation requirements dependent on the different delivery systems. By way of example, the pharmaceutical composition of the present invention may be formulated to be delivered using a mini-pump or by a mucosal route, for example, as a nasal spray or aerosol for inhalation or ingestable solution, or parenterally in which the composition is formulated by an injectable form, for delivery, by, for example, an intravenous, intramuscular or subcutaneous route. Alternatively, the formulation may be designed to be delivered by both routes.

Where the agent is to be delivered mucosally through the gastrointestinal mucosa, it should be able to remain stable during transit though the gastrointestinal tract; for example, it should be resistant to proteolytic degradation, stable at acid pH and resistant to the detergent effects of bile.

Where appropriate, the pharmaceutical compositions can be administered by inhalation, in the form of a suppository or pessary, topically in the form of a lotion, solution, cream, ointment or dusting powder, by use of a skin patch, orally in the form of tablets containing excipients such as starch or lactose, or in capsules or ovules either alone or in admixture with excipients, or in the form of elixirs, solutions or suspensions containing flavouring or colouring agents, or they can be injected parenterally, for example intravenously, intramuscularly or subcutaneously. For parenteral administration, the compositions may be best used in the form of a sterile aqueous solution which may contain other substances, for example enough salts or monosaccharides to make the solution isotonic with blood. For buccal or sublingual administration the compositions may be administered in the form of tablets or lozenges which can be formulated in a conventional manner.

For some embodiments, the agents of the present invention may also be used in combination with a cyclodextrin. Cyclodextrins are known to form inclusion and non-inclusion complexes with drug molecules. Formation of a drugcyclodextrin complex may modify the solubility, dissolution rate, bioavailability and/or stability property of a drug molecule. Drug-cyclodextrin complexes are generally useful for most dosage forms and administration routes. As an alternative to direct complexation with the drug the cyclodextrin may be used as an auxiliary additive, e. g. as a carrier, diluent or solubiliser. Alpha-, beta and gamma-cydodextrins are most commonly used and suitable examples are described in WO-A-91/11172, WO-A-94/02518 and WO-A-98/55148.

In a preferred embodiment, the agents of the present invention are delivered systemically (such as orally, buccally, sublingually), more preferably orally.

Hence, preferably the agent is in a form that is suitable for oral delivery.

It is to be understood that not all of the compounds need be administered by the same route. Likewise, if the composition comprises more than one active component, then those components may be administered by different routes.

The compounds of the invention may be milled using known milling procedures such as wet milling to obtain a particle size appropriate for tablet formation and for other formulation types. Finely divided (nanoparticulate) preparations of the compounds of the invention may be prepared by processes known in the art, for example see International Patent Application No. WO 02100196 (SmithKline Beecham).

For oral administration, the pharmaceutical compositions may take the form of, for example, tablets or capsules prepared by conventional means with pharmaceutically acceptable excipients such as binding agents (e.g. pregelatinised maize starch, polyvinylpyrrolidone or hydroxypropyl methylcellulose); fillers (e.g. lactose, microcrystalline cellulose or calcium hydrogen phosphate); lubricants (e.g. magnesium stearate, talc or silica); disintegrants (e.g. potato starch or sodium starch glycollate); or wetting agents (e.g. sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g. sorbitol syrup, cellulose derivatives or hydrogenated edible fats); emulsifying agents (e.g. lecithin or acacia); non-aqueous vehicles (e.g. almond oil, oily esters, ethyl alcohol or fractionated vegetable oils); and preservatives (e.g. methyl or propyl-p-hydroxybenzoates or sorbic acid). The preparations may also contain buffer salts, flavouring, colouring and sweetening agents as appropriate.

Preparations for oral administration may be suitably formulated to give controlled release of the active compound.

For buccal administration the composition may take the form of tablets or formulated in conventional manner.

The compounds of the invention may be formulated for parenteral administration by bolus injection or continuous infusion. Formulations for injection may be presented in unit dosage form e.g. in ampoules or in multl-dose containers, with an added preservative. The compositions may take such forms as suspensions, solutions or emulsions in oily or aqueous vehicles, and may contain formulatory agents such as suspending, stabilising and/or dispersing agents. Alternatively, the active ingredient may be in powder form for constitution with a suitable vehicle, e.g. sterile pyrogen-free water, before use.

The compounds of the invention may be formulated for topical administration in the form of ointments, creams, gels, lotions, pessaries, aerosols or drops (e.g. eye, ear or nose drops). Ointments and creams may, for example, be formulated with an aqueous or oily base with the addition of suitable thickening and/or gelling agents. Ointments for administration to the eye may be manufactured in a sterile manner using sterilised components.

Lotions may be formulated with an aqueous or oily base and will in general also contain one or more emulsifying agents, stabilising agents, dispersing agents, suspending agents, thickening agents, or colouring agents. Drops may be formulated with an aqueous or non-aqueous base also comprising one or more dispersing agents, stabilising agents, solubilising agents or suspending agents. They may also contain a preservative.

The compounds of the invention may also be formulated in rectal compositions such as suppositories or retention enemas, e.g. containing conventional suppository bases such as cocoa butter or other glycerides.

The compounds of the invention may also be formulated as depot preparations. Such long acting formulations may be administered by implantation (for example subcutaneously or intramuscularly) or by intramuscular injection. Thus, for example, the compounds of the invention may be formulated with suitable polymeric or hydrophobic materials (for example as an emulsion in an acceptable oil) or ion exchange resins, or as sparingly soluble derivatives, for example, as a sparingly soluble salt.

For intranasal administration, the compounds of the invention may be formulated as solutions for administration via a suitable metered or unitary dose device or alternatively as a powder mix with a suitable carrier for administration using a suitable delivery device.

A proposed dose of the compounds of the Invention is 1 to about 1000mg per day. It will be appreciated that it may be necessary to make routine variations to the dosage, depending on the age and condition of the patient and the precise dosage will be ultimately at the discretion of the attendant physician or veterinarian. The dosage will also depend on the route of administration and the particular compound selected.

Thus for parenteral administration a daily dose will typically be in the range of 1 to about 100 mg, preferably 1 to 80 mag per day. For oral administration a daily dose will typically be within the range 1 to 300 mg e.g. 1 to 100 mg.

### EXAMPLES

In the Intermediates and Examples unless otherwise stated:
All temperatures refers to °C. Infrared spectra were measured on a FT-IR instrument. Compounds were analysed by direct infusion of the sample dissolved in acetonitrile into a mass spectra operated in positive electro spray (ES⁺) ionisation mode. Proton Magnetic Resonance (¹H-NMR) spectra were recorded at 400 MHz, chemical shifts are reported in ppm downfield (d) from Me₄Si, used as internal standard, and are assigned as singlets (s), broad singlets (bs), doublets (d), doublets of doublets (dd), triplets (t), quartets (q) or multiplets (m). A strategy comprising of NOE (Nuclear Overhauser Effect) correlation and/or 1H,15N long range scalar correlations measurements has been implemented in order to allow elucidation of possible regio-isomers structure of compounds of the present invention. Proposed structures were verified by measurement of the vicinity in the space of key hydrogens, thus 1D Nuclear Overhauser difference spectra were used to measure 1H,1H-dipole-dipole correlations. In cases where NOE measurements were not conclusive, 1H,15N long range scalar correlations were measured via 1H,15N-HMBC experiments. A delay corresponding to an average long range scalar coupling 2,3J(1H,15N) of 6Hz was set for optimal result.

Column chromathography was carried out over silica gel (Merck AG Darmstaadt, Germany). The following abbreviations are used in the text: EtOAc = ethyl acetate, cHex = cyclohexane, CH₂CI₂ = dichloromethane, Et₂O = dietyl ether, DMF = N,N'-dimethylformamide, DIPEA = N,N-diisopropylethylamine, DME = ethylene glycol dimethyl ether, MeOH = methanol, Et₃N = triethylamine, TFA = trifluoroacetic acid, THF = tetrahydrofuran, DIBAI-H = diisobutylaluminium hydride, DMAP = dimethylaminopyridine, LHMDS = lithiumhexamethyldisilazane, KOtBu = potassium tert-butoxide, NMP = M-methyl-2-pyrrolidinone, MTBE = methyl-*tert*-butyl ether, IPA = isopropanol, DAST = (diethylamino)sulfur trifluoride, TMSBr = trimethylsilyl bromide, DDQ = 2,3-dichloro-5,6-dicyano-1,4-benzoquinone, SCX = strong cation exchanger, Tic refers to thin layer chromatography on silica plates, and dried refers to a solution dried over anhydrous sodium sulphate, r.t. (RT) refers to room temperature.

### Intermediate 1

### 6-Hydroxy-2-methyl-5-(2-propen-1-yl)-4(1H)-pvrimldinone

Sodium (2 g, 2.5 eq) was added portionwise to anh. MeOH (100 mL), at 0°C, under N₂. After consumption of metallic sodium, acetamidine hydrochloride (8.4 g, 2.5 eq) was added. After 10 min. of stirring the precipitated NaCI was filtered off. Diethyl-allyl-malonate (6 mL, 33 mmol) was added to the solution of free acetamidine and the mixture was stirred at r.t. for 2 days. The reaction mixture was concentrated and then neutralized with concentrated hydrochloric acid, filtered to obtain the title compound (4.25 g, 77%) as a white solid.
NMR (¹H, DMSO-d₆): δ 11.61 (bs, 2H), 5.75 (m, 1H), 4.92 (m, 1H), 4.84 (m, 1H), 2.94 (d, 2H), 2.19 (s, 3H).
MS (*m*/*z*): 166 (M)⁺.

### Intermediate 2

### 4,6-Dichloro-2-methyl-5-(2-propen-1-yl)pyrimidine

Intermediate 1 (6.0 g, 36.11 mmol) was mixed with POCl₃ (70 mL) and heated at reflux for 3 hr. The resulting solution was cooled to r.t. and poured slowly into ice/water (600 mL) with vigorous stirring. The product was extracted with EtOAc (3x50 mL). The combined organic extracts were washed with saturated NaHCO₃ (60 mL) and brine (40 mL), dried over anh. Na₂SO₄, filtered and concentrated *in vacuo.* The crude oil was purified by flash chromatography (silica gel, cHex 100%) to give the title compound (4.78 g, 65%) as a light yellow oil.
NMR (¹H, CDCl₃): δ 5.85 (m, 1H), 5.15 (dq, 1H), 5.11 (dq, 1H), 3.61 (dt, 2H), 2.67 (s, 3H).
MS (*m*/*z*): 202 [M]⁺.2Cl; 167 [MH-Cl]⁺,1Cl.

### Intermediate 3

### 6-Chloro-N-(2,4-dichlorophenyl)-2-methyl-5-(2-pronen-1-yl)-4-pyrimidinamine

A solution of 2,4-dichloroaniline (798 mg, 4.93 mmol) in anh. THF (22 mL), under N₂, was treated with sodium hydride 95%/oil(393 mg) at 0°C for 15 min before intermediate 2 (1 g) was added. The mixture was heated at reflux for 3 hr and quenched with water (20 mL). The product was extracted with ethyl acetate (2x20mL), dried over anh. Na₂SO₄ and concentrated *in vacuo.* The crude product was purified by flash chromatography (silica gel, EtOAc/cHex 4:96) to give the title compound (725 mg) as a white solid.
NMR (¹H, CDCl₃): δ 8.52 (d, 1H), 7.40 (d, 1H), 7.27 (dd, 1H), 7.21 (bs, 1H), 5.90 (m, 1H), 5.26 (m, 2H), 3.58 (m, 2H), 2.57 (s, 3H).
MS (*m*/*z*): 327 [M]⁺, 3Cl.

### Intermediate 4

### 1,1-Dimethylethyl [6-chloro-2-methyl-5-(2-propen-1-yl)-4-pyrimidinyl](2,4-dichlorophenyl)-carbamate

To a solution of intermediate 3 (146 mg, 0.444 mmol) in anh. CH₂Cl₂ (11 mL), under N₂, was added (Boc)₂O (194 mg, 2 eq) and DMAP (cat). The reaction mixture was stirred at r.t. for 18 hr. The solution was diluted with water (10 mL) and extracted with EtOAc (3x15 mL). The combined organic extracts were dried over anh. Na₂SO₄, filtered and concentrated to dryness *in vacuo*. Flash chromatography of the crude product (silica gel, cHex/EtOAc 95:5) gave the title compound (164 mg, 86%) as a colorless oil.
NMR (¹H, CDCl₃): δ 7.47 (d, 1H), 7.20 (dd, 1H), 7.17 (d, 1H), 5.75 (tq, 1H), 5.05(dd, 1 H), 4,97 (dd, 1H), 3.52 (d, 2H). 2.58 (s, 3H), 1,44 (s, 9H).
MS (*m*/*z*): 428 [MH]⁺, 3Cl; 372 [MH-tBu+H]⁺, 328 [MH-Boc+H]⁺

### Intermediate 5

### 1.1-Dimethylethyl [6-chloro-2-methyl-5-(2-oxoethyl)-4-pyrimidinyl](2,4-dichlorophenyl)-carbamate

A solution of intermediate 4 (300 mg, 0.670 mmol) in CH₂Cl₂ (10 mL) was ozonized (5g.h⁻1) at - 78°C for 10 min. When all the allyl pyrimidine had disappeared (TLC), the reaction mixture was first flushed with oxygen and then with nitrogen for 20 min. To the cooled reaction mixture was added (CH₃)₂S (258 µL, 5 eq) and the temperature was hallowed to warm up to 22°C. The solution was stirred for 18 hr at r.t.. The solvent was removed *in vacuo* and the crude product was purified by flash chromatography (silica gel, cHex/EtOAc 18.5:1.5) to give the title compound (250 mg, 86%) as a white solid.
NMR (¹H, CDCl₃): δ 9.59 (s, 1H), 7.77+7.57 (d+d, 1H), 7.47+7.37 (dd+dd, 1H), 7.47+7.41 (d+d, 1H), 3.83 (s, 2H), 2.46 (s, 3H), 1.33 (bs, 9H).
MS (*m*/*z*): 430 [MH]⁺.

### Intermediate 6

### 1,1-Dimethylethyl {6-chloro-2-methyl-5-[(2Z)-3-(methyloxy)-2-propen-1-yl]-4-pyrimidinyl}-(2,4-dichloroohenyl)carbamate

To a stirred suspension of (methoxy-methyl) triphenylphosphonium chloride (198 mg, 3 eq) in anh THF (3 mL) was added, at 0°C, under N₂, n-BuLi 1.6M in hexane (338 µL, 2.8 eq) dropwise. The mixture was allowed to stir for 10 min before a solution of intermediate 5 (83 mg, 0.193 mmol) in dry THF (1mL) was added. The reaction mixture was allowed to warm slowly to r.t. and left stirring for 3 hr. The mixture was quenched with water (5 mL) and extracted with EtOAc (3x10 mL). The combined organic extracts were dried over anh. Na₂SO₄, filtered and concentrated to dryness *in vacuo*.The crude product was purified by flash chromatography (silica gel, cHex/EtOAc 9:1) to give the title compound (39 mg, 44%) as a white solid.
NMR (¹H, CDCl₃): δ 7.50-7.47 (s, 1H), 7.28-7.15 (dd/d, 1+1H), 6.37-5.98 (d, 1H, Jₜᵣₐₙₛ=13Hz, J_{cis}=6Hz), 4.58-4.34 (m, 1H, Jₜᵣₐₙₛ=13Hz, J_{cis}=6Hz), 3.55-3.37 (d, 2H), 3.60-3.44(s, 3H), 2.58-2.53 (s, 3H), 1.55 (s, 9H).
MS (*m*/*z*): 458 [MH]⁺.

### Intermediate 7

### 1,1-Dimethylethyl [6-chloro-2-methyl-5-(3-oxopropyl)-4-pyrimidinyl](2,4-dichloronhenyl)-carbamate

Intermediate 6 (39 mg, 0.085 mmol) was stirred at r.t. with 4 mL of 4:1 THF-2N HCl for 78 hr. The mixture was then diluted with H₂O (4 mL) and extracted with EtOAc (4x5 mL). The combined organic extracts were dried over anh. Na₂SO₄, filtered and concentrated to dryness *in vacuo.* The crude product was purified by flash chromatography (silica gel, cHex/EtOAc 19:1) to give the title compound (26 mg, 69%) as a white solid.
NMR (¹H, CDCl₃): δ 9.83 (s, 1H), 7.45 (d, 1H), 7.3-7.2 (d/dd, 2H), 3.00 (m, 2H), 2.92 (m, 2H), 2.55 (s, 3H), 1.41 (s, 9H).
MS (*m*/*z*): 444[MH]⁺.

### Intermediate 8

### 1,1-Dimethylethyl [6-chloro-5-(3-hydroxypropyl)-2-methyl-4-pyrimidinyl](2,4-dichlorophenyl)carbamate

To a solution of intermediate 7 (21 mg, 0.047 mmol) in anh.CH₃OH (1 mL), at r.t., under N₂ was added NaBH₄ (7 mg, 4 eq). The reaction mixture was stirred for 1 hr. The solvent was removed *in vacuo* and the residue was redissolved in EtOAc (10 mL)/H₂O (10 mL), and the layers were separated. The aqueous layer was extracted with EtOAc (3x10 mL), and the combined organic extracts were dried over anh. Na₂SO₄, filtered, and concentrated *in vacuo* to afford the title compound (15 mg, 71%) as a white solid.
NMR (¹H, CDCl₃): δ 7.49 (m, 1H), 7.23 (m, 2H), 3.71 (m, 2H), 2.78 (m, 2H), 2.60 (s, 3H), 1.82 (m, 2H), 1.45 (s, 9H).
MS (*m*/*z*): 446 [MH]⁺.

### Intermediate 9

### 3-14-Chloro-6-((2,4-dichlorophenyl){[(1,1-dimethylethyl)oxy]carbonyl}amino)-2-methyl-5-pyrimidinyl]propyl methanesulfonate

To a solution of intermediate 8 (13 mg, 0.034 mmol) in anh. CH₂Cl₂ (1 mL), at r.t, under N₂, was added Et₃N (20 µl) and CH₃SO₂Cl (4 µl). The reaction was stirred at r.t. for 18 hr. The reaction mixture was diluted with water (5 mL) and extracted with EtOAc (3x5 mL). The combined organic extracts were dried over anh. Na₂SO₄, filtered and concentrated *in vacuo.* The crude product was purified by flash chromatography (silica gel, cHex/EtOAc 75/25) to give the title compound (25 mg, quantitative) as a white solid.
NMR (¹H, CDCl₃): δ 7.48 (t, 1H), 7.24 (m, 2H), 4.28 (t, 2H), 3.02 (s, 3H), 2.80 (m, 2H), 2.58 (s, 3H), 2.03 (m, 2H), 1.42 (s, 9H).

MS (*m*/*z*): 524 [MH]⁺.

### Intermediate 10

### 3-{4-Chloro-6-[(2,4-dichlorophenyl)amino]-2-methyl-5-pyrimidinyl}propyl methanesulfonate

A solution of intermediate 9 (50 mg, 0.095 mmol) in TFA 20%/CH₂Cl₂ (4 mL) was stirred at r.t. for 2 hr. The solvent was removed *in vacuo* and the residue was dissolved in EtOAc (10 mL) and saturated NaHCO₃ (10 mL), and the layers were separated. The aqueous layer was extracted with EtOAc (3x10 mL), and the combined organic extracts were dried over anh. Na₂SO₄, filtered and concentrated to dryness *in vacuo* to deliver the title compound (40 mg, 99%) as a white solid.
NMR (¹H, CDCl₃): δ 8.49 (d, 1H), 7.44 (d, 2H), 7.31 (dd, 2H), 7.22 (d, 1H), 4.39 (t, 2H), 3.05 (s, 3H), 2.93 (m. 2H), 2.56 (s, 3H), 2.13 (m, 2H).
MS (*m*/*z*): 424[MH]⁺.

### Intermediate 11

### 4-Chloro-8-(2,4-dichlorophenyl)-2-methyl-5,6,7,8-tetrahvdropyrido[2,3-d]pyrimidine

To a solution of intermediate 10 (40 mg, 0.094 mmol) in anh. THF (2mL) was added, at r.t., under N₂, NaH 95%ioil (5 mg, 2.1 eq). The reaction mixture was stirred for 2 hr at r.t.. The solution was diluted with water (8 mL) and extracted with EtOAc (2x8 mL). The combined organic extracts were dried over anh. Na₂SO₄, filtered and concentrated to dryness *in vacuo.* The crude product was purified by flash chromatography (silica gel, cHex/EtOAc 9:1) to give the title compound (25 mg, 81%) as a white solid.
NMR (¹H, CDCl₃): δ 7.51 (d, 1H), 7.32 (dd, 2H), 7.20 (d, 2H), 3.60 (m, 2H), 2.87 (m, 3H), 2.29 (s, 3H), 2.13 (m, 2H).

### Intermediate 12

### 2,4-Dichloro-1-(1-cyclohexen-1-yl)benzene

To a solution of *n*-butyl lithium 1.6M in hexanes (13.8 mL. 1.4 eq) in anh. THF (40 mL) at -78°C, under N₂, was added dropwise HN(iPr)₂ (3.32 mL, 1.5 eq). After stirring for 15 min, a solution of cyclohexanone (1.6 mL, 15.45 mmol) in anh. THF (4 mL) was added. After stirring for 15 min the enolate solution was warmed to room temperature and stirred for 2h. It was then cooled to -78°C and a solution of N-phenyl triflimide (6.1g, 17 mmol, 1.1 eq) in anh. THF (20 mL) was added to the enolate at -78°C. The reaction mixture was warmed to 0°C and stirred for 4h. The resulting solution was poured into water, the volume was reduced under vacuum and the residue was taken up in EtOAc and washed with H₂O (3x25 mL). The organic phase was dried over anh. Na₂SO₄. the solids were filtered and the solvent evaporated. The triflate was used as such in the following step. A mixture of the crude triflate obtained above (3g), 2,4-dichloro-benzeneboronic acid (3.2g, 1.1 eq), 1,1'-bis(diphenylphosphino-ferrocene)PdCl₂ (315mg, 0.025 eq) and K₂CO₃ (4.2 g, 2 eq), in toluene/acetone/water (26/26/6,5 mL) was heated at 80°C for 3h. The mixture was then treated with 1N NaOH (15 mL) and H₂O₂ 30% (10 mL) for 20 min at r.t. to reduce the residual borane. The product was extracted with toluene, washed with brine and dried over anh. Na₂SO₄. The solids were filtered, the solvent was evaporated and the residue was purified by flash chromatography (silica gel, 100% cHex). The title compound was obtained as a clear oil (2.18 g, 9.58 mmol, 62%) NMR (¹H, DMSO): δ 7.6 (d, 1H), 7.4 (dd, 1H), 7.27 (d, 1H), 5.69 (sett, 1H), 2.24 (m. 2H), 2.16 (m, 2H), 1.72 (m, 2H), 1.69 (m, 2H).
MS (*mlz*)*:* 226 [M]⁺.

### Intermediate 13

### 1-(2.4-Dichlorophenyl)-7-oxabicyclo[4.1.0]heptane

A solution of m-CPBA (2.25g, 3eq) in EtOAc (6 mL) was added dropwise to a stirred solution of intermediate 12 (1g, 4.4 mmol) in EtOAc (6 mL) at 0°C, under N₂. The reaction mixture was stirred at 0°C for 1h and at r.t. for 12h. When the reaction was complete (by t.l.c.) the reaction mixture was washed with 1N NaOH (3x10 mL) and H₂O (2x10 mL). It was then dried over anh. Na₂SO₄. the solids were filtered and the solvent evaporated. The title compound was obtained as a clear oil (940 mg, 3.87 mmols, 89%).
NMR (¹H, DMSO-d₆): δ 7.60 (m, 1H), 7.39 (m, 2H), 3.09 (t, 1H), 1.93 (m, 4H), 1.39 (m, 4H).
MS (*m*/*z*): 242 [M]⁺.

### Intermediate 14

### 2-(2,4-Dichtorophenyl)cyclohexanone

A solution of intermediate 13 (940 mg, 3.8 mmols) in EtOH (5 mL) was treated with conc. H₂SO₄ (1 mL) dissolved in H₂O (1 mL) and EtOH (5 mL). The solution was refluxed for 24 h and the EtOH was evaporated The residue was dissolved in EtOAc, washed with sat.aq. NaHCO₃ and H₂O. It was then dried over anh. Na₂SO₄, the solids were filtered and the solvent evaporated. The crude oil was purified by flash chromatography (silica gel, cHex/EtOAc 95:5). The title compound was obtained as a clear solid (345 mg, 1.42 mmol, 37%)
NMR (¹H, CDCl₃): δ 7.40 (d, 1H), 7.25 (dd, 1H), 7.15 (d, 1H). 4.06 (dd, 1H), 2.55 (m, 2H), 2.20 (m, 2H), 2.0-1.7 (m, 2H).
MS (*mlz*)*:* 242 [M]⁺.

### Intermediate 15

### Ethyl 3-(2,4-dichlorophenyl)-2-oxocyclohexanecarboxylate

To a suspension of NaH 80%/oil (48mg, 1.1 eq) in anh. THF (2mL), at 0°C, under N₂, was added intermediate 14 (350mg, 1.44mmol) dissolved in anh. THF (2.5 mL). After stirring for 15 min, to this solution was added dropwise a solution of LDA 0.5M/THF (3.2mL, 1.1 eq). After 15 min the solution was cooled to -78°C and ethyl cyanoformate (0.16mL, 1.1 eq) was added dropwise. The reaction mixture was stirred at -78°C for 20' and was then poured into water and ice and extracted with CH₂Cl₂ (3x10 mL). The combined organic extracts were dried over anh. Na₂SO₄, the solids were filtered and the solvent evaporated. The crude oil was purified by flash chromatography (silica gel, cHex/EtOAc 95:5). The title compound was obtained as a clear oil (150mg, 0.48 mmol, 33%) as a mixture of β-keto-ester and its enolic form in a 65:35 ratio.
NMR (¹H, DMSO-d₆): δ 12.18 (s, 1H), 7.55 (d, 1H),7.53 (d, 2H), 7.4-7.36 (dd, 2H), 7.32 (d+t, 2H), 7.23 (d, 1H), 4.23 (m, 2H), 4.11 (m, 2H), 4.03 (ta, 1H), 3.84 (ta, 1H), 2.4-1.8 (m, 12H), 1.27 (t+t, 6H).
MS (*m*/*z*): 314 [MH]⁺.

### Intermediate 16

### 8-(2,4-Dichlorophenyl)-2-methyl-5,6,7,8-tetrahvdro-4(1H)-guinazolinone

Sodium (20 mg, 1.84 eq) was added portionwise, under N₂, to anh. MeOH (2 mL). After consumption of metallic sodium, acetamidine hydrochloride (88 mg, 1.84 eq) was added to the solution. After 10 min the solid NaCl was filtered off and to the clear solution was added intermediate 15 (150 mg, 0.48 mmol) dissolved in anh. MeOH (2 mL). The reaction mixture was stirred at r.t. for 96 h and the solvent was then evaporated. The residue was purified by flash chromatography (silica gel, CH₂Cl₂/MeOH 95:5). The title compound was obtained as a white solid (92mg, 0.30 mmol, 62%).
NMR (¹H, CDCl₃): δ 11.2 (broad, 1H), 7.42 (d, 1H), 7.13 (dd, 1H), 6.72 (d, 1H), 4.34 (t, 1H), 2.65 (m, 1H), 2.56 (m, 1H), 2.34 (s, 3H), 2.07 (m, 1H), 1.92 (m, 1H), 1.72 (m, 1H), 1.65 (m, 1H).
MS (*m*/*z*): 309 [MH]⁺.

### Intermediate 17

### 4-Chloro-8-(2,4-dichlorophenyl)-2-methyl-5,6,7,8-tetrahydroquinazoline

Intermediate 16 (95 mg, 0.29 mmol) was dissolved in POCl₃ (3 mL) and the solution was refluxed for 3h. The POCl₃ was evaporated, the residue was dissolved in CH₂Cl₂ and poured into ice and cone. NH₄OH. The organic phase was separated and dried over anh. Na₂SO₄. The solids were filtered and the solvent evaporated. The crude product was purified by flash chromatography (silica gel, cHex/EtOAc 8:2) to obtain the title compound as a white solid (88mg, 0.27 mmol, 93%). NMR (¹H, CDCl₃): δ 7.42 (d, 1H), 7.13 (dd, 1H), 6.58 (d, 1H), 4.54 (t, 1H), 2.83 (m, 2H), 2.56 (s, 3H), 2.14 (m, 1H), 2.00 (m, 1H), 1.84 (m, 2H).
MS (*m*/*z*): 327 [MH]⁺.

### Intermediate 18

### Ethyl 2-chloro-6-methyl-4-[3-(2-oxoimidazolidin-1-yl)-1H-pyrazol-1-yl] nicotinate

To a solution of intermediate 33 (9.73 g, 1.5 eq) in anh. DMF (150 mL), at r.t., under N₂, was added NaH 60%/oil (1.7 g, 1 eq) and the reaction mixture was stirred at r.t. for 20 min. A solution of ethyl 2,4-dichloro-6-methyl-3-pyridinecarboxylate (10 g, 42.9 mmol) was then added dropwise and the reaction mixture was stirred at 80°C for 4 hr. It was then cooled down to r.t. and quenched with ice water. The addition of EtOAc caused a precipitate to form. The white solid was collected by filtration, washed with water and dried in vacuo (5.2 g). The filtrate was transferred into a separatory funnel and the aqueous layer was extracted with EtOAc (2x100 mL). The combined organic layers were washed with sat.aq. NaCl, dried over anh. Na₂SO₄, the solids were filtered and the solvent evaporated. The crude product was treated with EtOAc and left at r.t. overnight. The precipitate was filtrated, dried in vacuo and combined with the previous batch to give the title compound as a white solid (7.2 g, 48%).
NMR (¹H, DMSO-d₆): δ 8.53 (d, 1H), 7.77 (s, 1H), 7.18 (bs, 1H), 6.89 (d, 1H), 4.32 (q, 2H), 3.75 (t, 2H), 3.42 (t, 2H), 3.31 (s, 3H), 1.26 (t, 3H).
MS (*m*/*z*): 350 [MH]⁺.

### Intermediate 19

### 1-{1-[2-Chloro-3-(hydroxymethyl)-6-methyl-4-pyridinyl]-1H-pyrazol-3-yl}-2-imidazolidinone

To a suspension of intermediate 18 (7.2 g, 20.6 mmol) in anh. CH₂Cl₂ (120 mL), at 0°C, under N₂, was added dropwise DlBAl-H 1M/CH₂Cl₂, (41.2 mL, 2 eq). At the end of the addition the resulting solution was allowed to warm to r.t. and stirred for 2 hr. More DIBAI-H was added until the reaction was complete (3x20.5 mL), each time cooling at 0°C and then stirring at r.t. for 1 hr. The reaction mixture was then cooled to 0°C. quenched by the slow addition of a Rochelle salt solution (50 mL) and stirred at r.t. overnight. The white lattice was treated with 4 L of Roschell's salt solution and 3 L of CH₂Cl₂ and stirred at r.t. for 20 hr. The two phases were separated and the aqueous layer was extracted with CH₂Cl₂ (5x500 mL). The combined organic extracts were washed with sat.aq. NaCl, dried over anh. Na₂SO₄, the solids were filtered and the solvent evaporated to give the title compound as a white solid (4 g, 63%).
NMR (¹H, DMSO-d₆): δ 8.36 (d, 1H), 7.49 (s, 1H), 7.12 (bs, 1H), 6.86 (d, 1H), 5.47 (t, 1H), 4.61 (d, 2H), 3.88 (t, 2H), 3.44 (t, 2H), 3.30 (s, 3H).
MS (*mlz*): 308 [MH]⁺.

### Intermediate 20

### 1-{1-[2-Chtoro-3-(hydroxymethyl)-6-methyl-4-pyridinyl]-1H-pyridinyl]-1H-pyrazol-3-yl}-3-{[4-(methyloxy)-phenyl]methyl}-2-imidazolidinone

To a suspension of intermediate 19 (100 mg, 0.325 mmol) in anh. DMF (6.5 mL), at r.t., under N₂, was added NaH 60%/oil (13 mg, 1 eq.). The reaction mixture was stirred at r.t. until a pale yellow solution was obtained (*circa* 10 min). After cooling to 0°C, 1-(chloromethyl)-4-(methyloxy)benzene (44 µL, 1 eq) was added and the reaction mixture was stirred for 1.5 hr. It was partitioned between EtOAc/sat.aq. NaCl, the phases were separated and the organic layer was dried over anh. Na₂SO₄. The solids were filtered and the solvent evaporated. The crude product was purified by flash chromatography (silica gel, EtOAc/cHex 6:4 → 7:3) to give the title compound as a white solid (45.5 mg, 33%).
NMR (¹H, CDCl₃): δ 7.85 (d, 1H), 7.20 (dd, 2H), 7.15 (d, 1H), 7.10 (s, 1H), 6.89 (dd, 2H), 4.85 (s, 2H), 4.40 (s, 2H), 3.84 (t, 2H), 3.80 (s, 3H), 3.43 (t, 2H), 2.6 (s, 3H).
MS (m/z): 428 [MH]⁺.

### Intermediate 21

### 2-Chloro-6-methyl-4-[3-(3-{[4-(methyloxy)phenyl]methyl}-2-oxo-1-imidazolidinyl)-1H-pyrazol-1-yl]-3-pyridinecarbaldehyde

To a solution of intermediate 20 (925 mg, 2.16 mmol) in CH₂Cl₂ (90 mL), was added Dess Martin periodinane (1.38 g, 1.5 eq) in three portions and the reaction mixture was stirred at r.t. for 2 hr. More Dess Martin periodinane (750 mg, 0.2 eq) was added and the reaction mixture was stirred for an additional 30 min. Na₂S₂O₃ (5 eq) in a sat.aq. NaHCO₃ (100 mL) was added and the phases were separated. The aqueous layer was extracted with CH₂Cl₂ (3x50 mL) and the combined organic extracts were dried over anh. Na₂SO₄, the solids were filtered and the solvent evaporated. The crude product was purified by flash chromatography (silica gel, EtOAc/cHex 6:4 → 7:3) to give the title compound as a white solid (520 mg, 57%).
NMR (¹H, CDCl₃): δ 10.26 (s, 1H), 7.85 (d, 1H), 7.23 (dd, 2H), 7.20 (s, 1H), 7.13 (d, 1H), 6.89 (dd, 2H), 4.41 (s, 2H), 3.84 (t, 2H), 3.80 (s, 3H), 3.39 (t, 2H), 2.6 (s, 3H).
MS (m/z): 426 [MH]⁺.

### Intermediate 22

### 1-(1-{2-Chloro-6-methyl-3-[(E)-2-(methyloxy)ethenyl]-4-pyridinyl}-1H-pyrazol-3yvl)-3-{[4-(methyloxy)phenyl]methyl}-2-imidazolidinone

n-BuLi 1.6M/Hexane (0.44 mL, 3 eq) was added dropwise to a suspension of (methoxymethyl)-triphenylphosphonium chloride (224 mg, 3 eq) in anh. THF (5 mL) at 0°C, under N₂. At the end of the addition the reaction mixture was allowed to warm to r.t. and stirred for 20 min. A solution of intermediate 21 (100 mg, 0.235 mmol) in THF (8 mL) was added and the reaction mixture stirred at r.t. for an additional 1.5 hr. The mixture was treated with water, EtOAc was added and the phases were separated. The organic layer was dried over anh. Na₂SO₄, the solids were filtered and the solvent evaporated in vacuo to a residue which was purified on an SCX cartridge (100% cHex → cHex/EtOAc 7:3) to give the title compound as a white solid (68 mg, 63%) as a 7:3 mixture of trans:cis isomers.
NMR (¹H, CDCl₃): δ 7.36 (d, 1H), 7.24 (m, 3H), 6.99 (d, 1H), 6.87 (d, 2H), 6.58 (d, 2H), 5.59 (d, 2H), 4.40 (s, 2H), 3.89 (m, 2H), 3.78 (s, 3H), 3.64 (s, 3H), 3.37 (m, 2H), 2.50 (s, 3H).
MS (m/z): 454 [MH]⁺.

### Intermediate 23

### {2-Chloro-6-methyl-4-[3-(3-{[4-(methyloxy)phenyl]methyl}-2-oxo-1-imidazolidinyl)-1H-pyrazol-1-yl]-3-pyridinyl}acetaidehyde

To a solution of intermediate 22 (5.5 g, 12.5 mmol) in THF (120 mL), at r.t., was added 6.0M HCl (60 mL, 28.4 eq.) and the reaction mixture was stirred for 18 hr. The reaction mixture was quenched with sat.aq. NaHCO₃ untill neutral pH, the solvent partially removed and the crude mixture partitioned between EtOAc/water. The phases were separated and the organic layer was washed with sat.aq. NaCl (2x10 mL). It was dried over anh. Na₂SO₄, the solids were filtered and the solvent evaporated. The crude product was purified by flash chromatography (silica gel, cHex/EtOAc 4:6) to give the title compound as a white solid (4.6 g, 86%).
NMR (¹H, CDCl₃): δ 9.73 (s, 1H), 7.70 (d, 1H), 7.23 (d, 2H), 7.06 (m, 2H), 6.88 (d, 2H), 4.40 (s, 2H), 4.01 (s, 2H), 3.80 (s, 3H), 3.76 (t, 2H), 3.37 (t, 2H), 2.58 (s, 3H).
MS (m/z): 440 [MH]⁺.

### Intermediate 24

### 1-(1-{2-chloro-6-methyl-3-[(2E)-3-(methyloxy)-2-propen-1-yl]-4-pyridinyl}-1H-pyrazol-3-yl)-3-{[4-(methyloxy)phenyl]methyl}-2-imidazolidinone

*n*-BuLi 1.6M/Hexane (3 eq) may be added dropwise to a suspension of (methoxymethyl)-triphenylphosphonium chloride (3 eq) in anh. THF, at 0°C, under N₂. At the end of the addition the reaction mixture may be allowed to warm to r.t. and stirred for 20 min. A solution of intermediate 23 in anh. THF may be added and the reaction mixture stirred at r.t. for an additional 1.5 hr. The mixture may be treated with water, EtOAc may be added and the phases separated. The organic layer may be dried over anh. Na₂SO₄, the solids filtered and the solvent evaporated in vacuo to a residue which may be purified on an SCX cartridge to give the title compound as a mixture of trans:cis isomers.

### Intermediate 25

### 3-12-chloro-6-methyl-4-13-(3-{[4-(methyloxy)phenyl]methyl}-2-oxo-1-imidazolidinyl)-1H-pyrazol-1-yl]-3-pyridinyl}propanal

To a solution of intermediate 24 in THF, at r.t., may be added 6.0M HCl (large excess) and the reaction mixture may be stirred at r.t. for 18 hr. The reaction mixture may be quenched with sat.aq. NaHCO₃ untill neutral pH, the solvent partially removed and the crude mixture partitioned between EtOAc/water. The phases may be separated and the organic layer washed with sat.aq. NaCl (2x). It may be dried over anh. Na₂SO₄, the solids filtered and the solvent evaporated. The crude product may be purified by flash chromatography (silica gel) to give the title compound.

### Intermediate 26

### 1-{1-[2-chloro-3-(3-hydroxypropyl)-6-methyl-4-pyridinyl]-1H-pyrazol-3-yl}-3-{[4-(methyloxy)phenyl]methyl)-2-imidazolidinone

To a solution of intermediate 25 (4.4 g, 9.96 mmol) in anh. MeOH (100 mL) at 0°C, under N₂, was added NaBH₄ (397 mg, 1.0 eq) in small portions and the reaction mixture was warmed up to r.t. and stirred for 30 min. The reaction mixture was quenched with water, the solvent partially removed and partitioned between EtOAc/water. The phases were separated and the organic layer was washed with sat.aq. NaCl (2x10 mL). It was dried over anh. Na₂SO₄, the solids were filtered and the solvent evaporated. The crude product was purified by flash chromatography (silica gel, cHex/EtOAc1:1) to give the title compound as a white solid (4.26 g, 96%).

### Intermediate 27

### 1-{1-[2-chloro-3-(3-{[(1,1-dimethyletyyl)(dimethyl)silyl]oxy}propyl)-6-methyl-4-pyridinyl]-1H-pyrazol-3-yl}-3-{[4-(methyloxy)phenyl]methyl}-2-imidazolidinone

To a solution of intermediate 26 in anh. DMF, at 0°C, under N₂, may be added imidazole (11 eq), DMAP (0.1 eq) and *tert*-butyldimethylsilyl chloride (2.8 eq). The reaction mixture may be warmed up to r.t. and stirred for 1 hr. It may then be partitioned between EtOAc/sat.aq. NH₄Cl and the phases separated. The organic layer may be washed with sat.aq. NaCl (2x) and dried over anh. Na₂SO₄. The solids may be filtered and the solvent evaporated. The crude product may be purified by flash chromatography (silica gel) to give the title compound.

### Intermediate 28

### 1-{1-[2-[(2,4-dichlorophenyl)amino]-3-(3-{[(1,1-dimethylethyl)(dimethyl)silyl]oxy}propyl)-6-methyl-4-pyridinyl]-1H-pyrazol-3-yl}-3-{[4-(methyloxy)phenyl]methyl}-2-imidazolidinone

To a solution of intermediate 27 in anh. DME, at r.t., under N₂, may be added Pd₂(dba)₃ (0.1 eq), dicyclohexyl(2'-methyl-2-biphenylyl)phosphane (0.3 eq), K₃PO₄ (3 eq) and 2,4-dichloroaniline (1.5 eq) and the reaction mixture may be stirred and heated at reflux for 3 hr. It may then be partitioned between EtOAc/sat.aq. NH₄Cl, the phases separated and the organic layer washed with sat.aq. NaCl (2x). It may be dried over anh. Na₂SO₄. the solids filtered and the solvent evaporated. The crude product may be purified by flash chromatography (silica gel) to give the title compound.

### Intermediate 29

### 1-{1-[2-[(2,4-dichlorophenyl)amino]-3-(3-hydroxypropy)-6-methyl-4-pyridinyl]-1H-pyrazol-3-yl}-3-{[4-(methyloxy)phenyl]methyl}-2-imidazolidinone

To a solution of intermediate 28 in anh. THF, at r.t., under N₂, may be added Et₃N·3HF (5 eq) and the reaction mixture may be stirred at r.t. for 18 hr. It may be partitioned between EtOAc/water, the phases separated and the organic layer washed with sat.aq. NaCl (2x). It may be dried over anh. Na₂SO₄, the solids filtered and the solvent evaporated. The crude product may be purified by flash chromatography (silica gel) to give the title compound.

### Intermediate 30

### 1-{1-[8-(2,4-dichlorophenyl)-2-methyl-5,6,7,8-tetrahydro-1,8-naphthyridin-4-yl]-1H-pyrazol-3-yl}-3-{[4-(methyloxy)phenyl]methyl}-2-imidazolidinone

To a solution of intermediate 29 in anh. CH₂Cl₂, under N₂, may be added I₂ (2 eq), PPh₃ (2 eq) and Et₃N (2 eq) and the reaction mixture may be stirred at r.t. for 2 hr. The solvent may be evaporated and the crude product purified on an SCX cartridge and by flash chromatography (silica gel) to give the title compound.

### Intermediate 31

### N-(2-Chloroethyl)-3-({[(2-chloroethyl)amino]carbonyl}amino)-1H-pyrazole-1-carboxamide

To a solution of 3-aminopyrazole (500 mg, 6 mmol) in anh. DMF (3 mL), at 0°C, under N₂, was added 3-chloroethyl isocyanate (1.53 mL, 3 eq) and the reaction mixture was stirred at r.t. for 2 hr, after which the solvent was evaporated. The crude product was purified by flash chromatography (silica gel, cHex/EtOAc 1:1) to give the title compound (1.593 g, 89%).
NMR (¹H, DMSO): δ 9.20 (s, 1H), 8.26 (m, 1H), 8.10 (d, 1H), 7.25 (bs, 1H), 6.37 (d, 1H), 3.74 (m, 2H), 3.66 (m, 2H), 3.58 (m, 2H), 3.46 (m, 2H).
MS (m/z): 296 [MH]⁺.

### Intermediate 32

### N-(2-Chloroethyl)-3-(2-oxoimidazolidin-1-yl)-1H-pyrazole-1-carboxamide

To a solution of intermediate 31 (100 mg, 0.34 mmol) in anh. THF (4 mL), at r.t., under N₂, was added KOt-Bu (42 mg, 1.1 eq) and the reaction mixture was stirred for 2 hr. Water (0.5 mL) was added and the solvent was evaporated. The aqueous phase was diluted with H₂O and extracted with EtOAc (3 x 20 mL). The combined organic extracts were dried over anh. Na₂SO₄. The solids were filtered and the solvent evaporated. The crude product was purified by flash chromatography (silica gel, EtOAc/cHex 8:2, then 9:1) to give the title compound as a white solid (39 mg, 44%) NMR (¹H, DMSO): δ 8.18 (bt, 1H), 8.11 (d, 1H), 7.14 (bs, 1H), 6.75 (d, 1H), 3.89 (m, 2H), 3.73 (m, 2H), 3.56 (m, 2H), 3.40 (m, 2H).
MS (m/z): 258 [MH]⁺.

### Intermediate 33

### 1-(1H-Pyrazol-3-yl)imidazolidin-2-one

To a solution of intermediate 32 (190 mg, 0.74 mmol) in a 2:1 mixture of MeOH/H₂O (15 mL), at r.t., under N₂, was added LiOH (177 mg, 10 eq) and the reaction mixture was heated at 80°C for 3 hr. It was cooled down to r.t. and neutralized to pH 7 with 2M HCl. Silica gel was then added and the solvents were evaporated. The adsorbed crude product was purified by flash chromatography (silica gel. EtOAc/MeOH 9:1) to give the title compound as a white solid (80 mg, 71%)
NMR (¹H, DMSO): δ 12.10 (bs, 1H), 7.6 (s, 1H), 6.7 (s, 1H), 6.4 (s, 1H), 3.8 (t, 2H), 3.4 (t, 2H). MS (m/z): 152 [MH]⁺.

### EXAMPLE 1

### Synthesis of representative compounds of structure (Ia) in which Y = N

### Example 1-1

### 1-{1-[8-(2,4-dichlorophenyl)-2-methyl-5,6,7,8-tetrahydropyrido[2.3-d]avrimidin-4-yl]-1H-pyrazol-3-yl}-2-imidazolidinone

To a solution of intermediate 33 (2 eq) in anh. DMF, at r.t., under N₂, may be added NaH 60%/oil (2 eq). The reaction mixture may be stirred at r.t. for 20 min. A solution of intermediate 11 in anh. DMF may be added and the reaction mixture may be heated at 80°C for 5 hr. Water and EtOAc may be added and the phases separated. The aqueous layer may be further extracted with EtOAc (2x). The combined organic extracts may be dried over anh. Na₂SO₄, the solids filtered and the solvent evaporated. The residue may be purified by flash chromatography (silica gel) to give the title compound.

### EXAMPLE 2

### Synthesis of representative compounds of structure (Id)

### Example 2-1

### 1-{1-[8-(2,4-dichlorophenyl)-2-methyl-5,6,7,8-tetrahydro-4-quinazolinyl]-1H-pyrazol-3-yl}-2-imidazolidinone

To a solution of intermediate 33 (2 eq) in anh. DMF, at r.t., under N₂, may be added NaH 60%/oil (2 eq). The reaction mixture may be stirred at r.t. for 20 min. A solution of intermediate 17 in anh. DMF may be added and the reaction mixture may be heated at 80°C for 5 hr. Water and EtOAc may be added and the phases separated. The aqueous layer may be further extracted with EtOAc (2x). The combined organic extracts may be dried over anh. Na₂SO₄, the solids filtered and the solvent evaporated. The residue may be purified by flash chromatography (silica gel) to give the title compound.

### EXAMPLE 3

### Synthesis of representative compounds of structure (Ia) in which Y = -CH

### Example 3-1

### 1-{1-[8-(2,4-dichlorophenyl)-2-methyl-5,6,7,8-tetrahydro-1,8-naphthyridin-4-yl]-1H-pyrazol-3-yl}-2-imidazolidinone

To a solution of intermediate 30 in TFA, under N₂, may be added anisole (10 eq) and the reaction mixture stirred and heated at 80°C for 2 hr. It may be cooled down to r.t., the TFA evaporated and the reaction mixture partitioned between CH₂Cl₂/sat.aq. NaHCO₃. The phases may be separated and the organic layer washed with sat. aq. NaCl (2x). It may be dried over anh. Na₂SO₄, the solids filtered and the solvent evaporated. The crude product may be purified by flash chromatography (silica gel) to give the title compound.

### EXAMPLE 4

### CRF Binding Activity

CRF binding affinity may be determined in vitro by the compounds' ability to displace ¹²⁵I-oCRF and ¹²⁵I-Sauvagine for CRF1 and CRF2 SPA, respectively, from recombinant human CRF receptors expressed in Chinese Hamster Ovary (CHO) cell membranes. For membrane preparation, CHO cells from confluent T-flasks may be collected in SPA buffer (HEPES/KOH 50mM, EDTA 2mM; MgCl₂ 10mM, pH 7.4.) in 50mL centrifuge tubes, homogenized with a Polytron and centrifuged (50'000g for 5min at 4°C: Beckman centrifuge with JA20 rotor). The pellet may be resuspended, homogenized and centrifuged as before.

The SPA experiment may be carried out in Optiplate by the addition of 100 µL the reagent mixture to 1µL of compound dilution (100% DMSO solution) per well. The assay mixture may be prepared by mixing SPA buffer, WGA SPA beads (2.5 mg/mL), BSA (1 mg/mL) and membranes (50 and 5 µg of protein/mL for CRF1 and CRF2 respectively) and 50 pM of radioligand.

The plate may be incubated overnight (>18 hrs) at room temperature and read with the Packard Topcount with a WGA-SPA ¹²⁵I counting protocol.

### EXAMPLE 5

### CRF functional assay

Compounds of the invention may be characterised in a functional assay for the determination of their inhibitory effect. Human CRF-CHO cells may be stimulated with CRF and the receptor activation was evaluated by measuring the accumulation of cAMP.

CHO cells from a confluent T-flask were resuspended with culture medium without G418 and dispensed in a 96-well plate, 25'000c/well, 100 µLlwell and incubated overnight. After the incubation the medium may be replaced with 100 µL of cAMP IBMX buffer warmed at 37°C (5mM KCl, 5mM NaHCO₃, 154mM NaCl, 5mM HEPES, 2.3mM CaCl₂, 1mM MgCl_{2;} 1g/L glucose, pH 7.4 additioned by 1 mg/mL BSA and 1mM IBMX) and 1µL of antagonist dilution in neat DMSO. After 10 additional minutes of incubation at 37°C in a plate incubator without CO2, 1µL of agonist dilution in neat DMSO may be added. As before, the plate may be incubated for 10 minutes and then cAMP cellular content may be measured by using the Amersham RPA 538 kit.

## Claims

1. Compounds of formula (Ia) including stereoisomers and pharmaceutically acceptable salts or solvates thereof 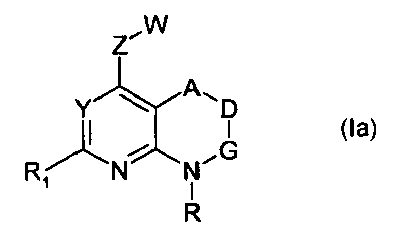 wherein
R is an aryl group selected from: 2,4-dichlorophenyl, 2-chloro- 4-methylphenyl, 2-chloro-4-trifluoromethylphenyl, 2-chloro-4- methoxyphenyl, 2,4,5-trimethylphenyl, 2,4-dimethylphenyl, 2- methyl-4-methoxyphenyl, 2-methyl-4-ethoxyphenyl, 2- methyl-4-isopropoxyphenyl, 2-methyl-4-hydroxyphenyl, 2- methyl-4-chlorophenyl, 2-methyl-4-trifluoromethylphenyl, 2,4- dimethoxyphenyl, 2-methoxy-4-trifluoromethylphenyl, 2- methoxy-4-chlorophenyl, 3-methoxy-4-chlorophenyl, 2,5- dimethoxy-4-chlorophenyl, 2-methoxy-4-isopropylphenyl, 2- methoxy-4-trifluoromethylphenyl, 2-methoxy-4- isopropylphenyl, 2-methoxy-4-methylphenyl, 2- trifluoromethyl-4-chlorophenyl, 2,4-bis-trifluoromethylphenyl, 2-trifluoromethyl-4-methylphenyl, 2-trifluoromethyl-4- methoxyphenyl, 2-difluoromethyl-4-methoxyphenyl, 2-bromo- 4-isopropylphenyl, 2-methyl-4-cyanophenyl, 2-chloro-4- cyanophenyl, 2-trifluoromethyl-4-cyanophenyl, 2- trifluoromethoxy-4-cyanophenyl, 2-ethyl-4-cyanophenyl, 2- methyl-4-trifluoromethoxyphenyl, 4-methyl-6-dimethyl- aminopyridin-3-yl, 2,6-bismethoxy-pyridin-3-yl, 2-methyl-6- methoxy-pyridin-3-yl, 2-trifluoromethyl-6-methoxy-pyridin-3- yl, 3-chloro-5-trichloromethyl-pyridin-2-yl, 2-methyl-4- (pyrazol-1-yl)-phenyl, 2-methoxy-4-(pyrazol-1-yl)-phenyl, 2,4,6-trimethoxyphenyl, 2-methyl-4,5-benzodioxolyl, 2- methyl-3,4-benzodioxolyl;
R₁ is -CH₃;
R₃ is hydrogen or C1-C6 alkyl;
R₄ is hydrogen or C1-C6 alkyl;
R₅ is a C1-C6 alkyl, halo C1-C6 alkyl, C1-C6 alkoxy, halo C1- C6 alkoxy, C3-C7 cycloalkyl, hydroxy, halogen, nitro, cyano, -NR₃R₄;
R₇ is hydrogen;
R₈ is hydrogen;
R₉ is hydrogen;
R₁₀ is hydrogen;
R₁₁ is hydrogen;
R₁₂ is hydrogen;
R₁₃ is hydrogen;
R₁₄ is R₃;
D is CR₈R₉;
G is CR₁₀R₁₁ or is CR₁₀;
A is CR₁₂R₁₃;
X is carbon or nitrogen;
Y is nitrogen or -CR₇;
W is 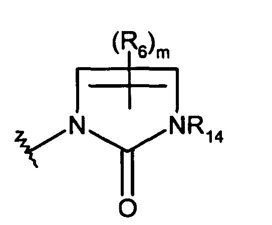
Z is pyrazole which may be substituted by 1 to 2 R₅ groups;
m is 0.

2. Compounds according to claim 1, selected from the following group:
1-{1-[8-(2,4-dichlorophenyl)-2-methyl-5,6,7,8-tetrahydropyrido[2,3-d]pyrimidin-4-yl]-1H-pyrazol-3-yl}-2-imidazolidinone;
1-{1-[8-(2,4-dichlorophenyl)-2-methyl-5,6,7,8-tetrahydro-1,8-naphthyridin-4-yl]-1H-pyrazol-3-yl}-2-imidazolidinone.

3. Process for preparing compounds of formula (Ia) comprising the following steps: 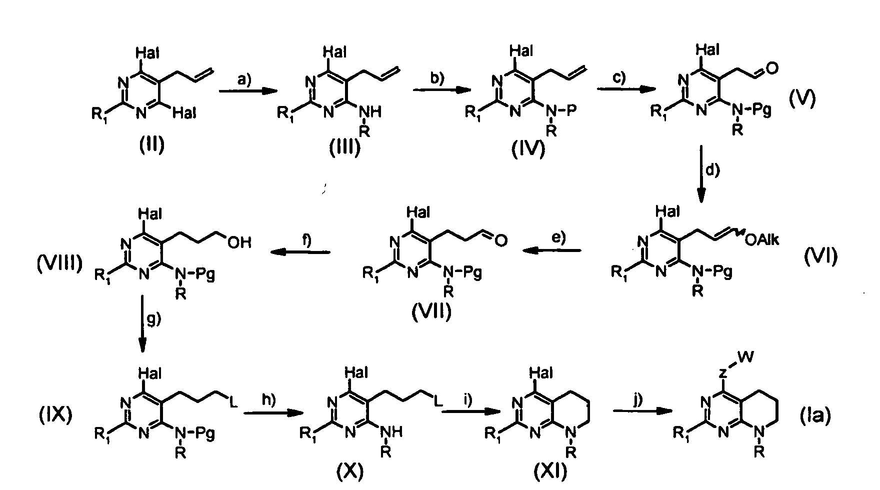 in which
step a stands for the nucleophilic substitution with a suitable amine (such as a substituted aniline) of compounds of formula (II), in basic conditions (such as sodium hydride in a polar aprotic solvent) to give compounds (III);
step b stands for the protection of the amino group with a suitable protecting group (such as a BOC group);
step c stands for the oxidation of the double bond with a suitable oxidizing agent (such as ozone in a polar protic solvent) to give the aldehyde of compounds (V);
step d + e stands for formation of the aldehyde group of compounds (VII) through formation of the enol ether by Wittig reaction in the usual conditions, followed by acid hydrolysis (step e);
step f stands for the reduction of the aldehyde group of compounds (VII) to the alcohol of compounds (VIII) with a suitable reducing agent (such as sodium borohydride);
step g stands for the conversion of the alcohol of compounds (VIII) into a suitable leaving group (such as, for example, a halogen or reactive residue of sulphonic acid (e.g. mesylate, tosylate), preferably mesylate);
step h stands for the deprotection of the amino group of compounds (IX);
step i stands for the intramolecular cyclization to give the cyclized compounds (X)
step j stands for conversion of the halogen derivative, preferably chloride, into compounds (Ia), by reaction with the suitable reactive -Z-W derivative, in basic conditions (such as, for example, sodium hydride in a polar solvent).

4. The use of a compound according to anyone from claim 1 to 2, in the preparation of a medicament for use in the treatment of conditions mediated by CRF (corticotropin-releasing factor).

5. The use of a compound according to claim 4, in the preparation of a medicament for use in the treatment of depression and anxiety.

6. The use of a compound according to claim 4, in the preparation of a medicament for use in the treatment of IBS (irritable bowel disease) and IBD (inflammatory bowel disease).

7. A compound according to anyone from claim 1 to 2, for the treatment of conditions mediated by CRF (corticotropin-releasing factor).

8. A pharmaceutical composition comprising a compound of anyone from claim 1 to 2, in admixture with one or more physiologically acceptable carriers or excipients.

## Patentansprüche

1. Verbindungen der Formel (Ia), einschließlich Stereoisomere und pharmazeutisch
verträgliche Salze oder Solvate davon 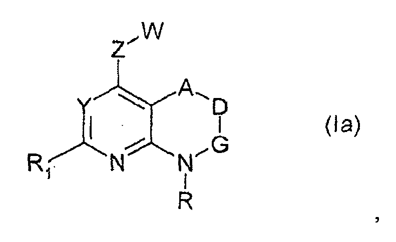 wobei
R ein Arylrest ist, ausgewählt aus: 2,4-Dichlorphenyl, 2-Chlor-4-methylphenyl, 2-Chlor-4-trifluormethylphenyl, 2-Chlor-4-methoxyphenyl, 2,4,5-Trimethyl- phenyl, 2,4-Dimethylphenyl, 2-Methyl-4-methoxyphenyl, 2-Methyl-4-ethoxy- phenyl, 2-Methyl-4-isopropoxyphenyl, 2-Methyl-4-hydroxyphenyl, 2-Methyl-4- chlorphenyl, 2-Methyl-4-trifluormethylphenyl, 2,4-Dimethoxyphenyl, 2-Methoxy-4-trifluormethylphenyl, 2-Methoxy-4-chlorphenyl, 3-Methoxy-4- chlorphenyl, 2,5-Dimethoxy-4-chlorphenyl, 2-Methoxy-4-isopropylphenyl, 2-Methoxy-4-trifluormethylphenyl, 2-Methoxy-4-isopropylphenyl, 2-Methoxy- 4-methylphenyl, 2-Trifluormethyl-4-chlorphenyl, 2,4-Bis-trifluonnethylphenyl, 2-Trifluormethyl-4-methylphenyl, 2-Trifluonnethyl-4-methoxyphenyl, 2-Difluormethyl-4-methoxyphenyl, 2-Brom-4-isopropylphenyl, 2-Methyl-4- cyanophenyl, 2-Chlor-4-cyanophenyl, 2-Trifluormethyl-4-cyanophenyl, 2-Trifluonnethoxy-4-cyanophenyl, 2-Ethyl-4-cyanophenyl, 2-Methyl-4- trifluormethoxyphenyl, 4-Methyl-6-dimethylaminopyridin-3-yl, 2,6-Bismethoxy- pyridin-3-yl, 2-Methyl-6-methoxypyridin-3-yl, 2-Trifluormethyl-6- methoxypyridin-3-yl, 3-Chlor-5-trichlormethylpyridin-2-yl, 2-Methyl-4- (pyrazol-1-yl)phenyl, 2-Methoxy-4-(pyrazol-1-yl)phenyl, 2,4,6-Trimethoxy- phenyl, 2-Methyl-4,5-benzodioxolyl, 2-Methyl-3,4-benzodioxolyl;
R₁ -CH₃ ist;
R₃ Wasserstoff oder C1-C6-Alkyl ist;
R₄ Wasserstoff oder C1-C6-Alkyl ist;
R₅ ein C1-C6-Alkyl, Halogen-C1-C6-alkyl, C1-C6-Alkoxy, Halogen-C1-C6- alkoxy, C3-C7-Cycloalkyl, Hydroxy, Halogen, Nitro, Cyano, -NR₃R₄ ist;
R₇ Wasserstoff ist;
R₈ Wasserstoff ist;
R₉ Wasserstoff ist;
R₁₀ Wasserstoff ist;
R₁₁ Wasserstoff ist;
R₁₂ Wasserstoff ist;
R₁₃ Wasserstoff ist;
R₁₄ R₃ ist;
D CR₈R₉ ist;
G CR₁₀R₁₁ ist oder CR₁₀ ist;
A CR₁₂R₁₃ ist;
Y Stickstoff oder -CR₇ ist;
W 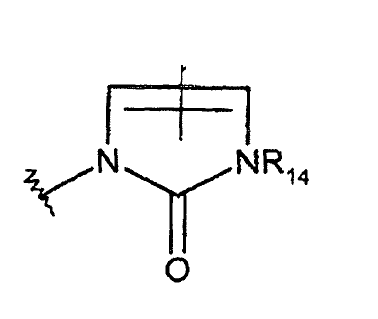 ist;
Z Pyrazol ist, welches mit 1 bis 2 Resten R₅ substituiert sein kann.

2. Verbindungen gemäß einer von Anspruch 1, ausgewählt aus der folgenden Gruppe:
1-{1-[8-(2,4-Dichlorphenyl)-2-methyl-5,6,7,8-tetrahydropyrido[2,3-d]pyrimidin-4-yl]-1H-pyrazol-3-yl}-2-imidazolidinon;
1-{1-[8-(2,4-Dichlorphenyl)-2-methyl-5,6,7,8-tetrahydro-1,8-naphthyridin-4-yl]-1H-pyrazol-3-yl}-2-imidazolidinon.

3. Verfahren zur Herstellung von Verbindungen der Formel (Ia), umfassend die folgenden Schritte: 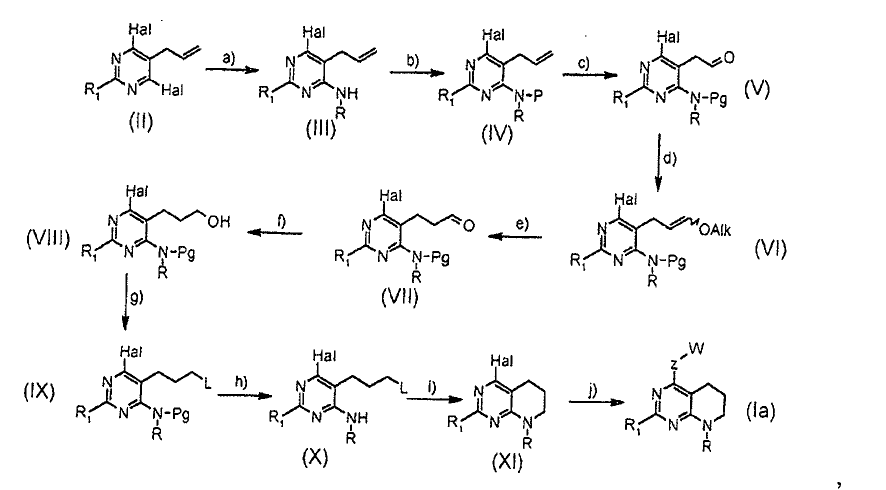 wobei
Schritt a für die nukleophile Substitution mit einem geeigneten Amin (wie einem substituierten Anilin) von Verbindungen der Formel (II) unter basischen Bedingungen (wie Natriumhydrid in einem polaren aprotischen Lösungsmittel), um die Verbindungen (III) zu ergeben, steht;
Schritt b für den Schutz der Aminogruppe mit einer geeigneten Schutzgruppe (wie einem BOC-Rest) steht;
Schritt c für die Oxidation der Doppelbindung mit einem geeigneten Oxidationsmittel (wie Ozon in einem polaren protischen Lösungsmittel), um den Aldehyd der Verbindungen (V) zu ergeben, steht;
Schritt d + e für die Bildung der Aldehydgruppe der Verbindungen (VII) durch Bildung des Enolethers durch eine Wittig-Reaktion unter den üblichen
Schritt f Bedingungen, gefolgt von Säurehydrolyse (Schritt e), steht; für die Reduktion der Aldehydgruppe der Verbindungen (VII) zum Alkohol der Verbindungen (VIII) mit einem geeigneten Reduktionsmittel (wie Natriumborhydrid) steht;
Schritt g für die Umwandlung des Alkohols der Verbindungen (VIII) in eine geeignete Abgangsgruppe (wie zum Beispiel ein Halogen oder ein reaktiver Rest der Sulfonsäure (z.B. Mesylat, Tosylat), bevorzugt Mesylat), steht;
Schritt h für die Entschützung der Aminogruppe der Verbindungen (IX) steht;
Schritt i für die intramolekulare Cyclisierung, um die cyclisierten Verbindungen (X) zu ergeben, steht;
Schritt j für die Umwandlung des Halogenderivats, bevorzugt Chlorids, in die Verbindungen (Ia) durch Umsetzung mit dem geeigneten reaktiven -Z-W-Derivat unter basischen Bedingungen (wie zum Beispiel Natriumhydrid in einem polaren Lösungsmittel) steht.

4. Die Verwendung einer Verbindung gemäß einer von Anspruch 1 bis 2 zur Herstellung eines Medikaments zur Verwendung bei der Behandlung von durch CRF (Corticoliberin) vermittelten Zuständen.

5. Die Verwendung einer Verbindung gemäß Anspruch 4 zur Herstellung eines Medikaments zur Verwendung bei der Behandlung von Depression und Angst.

6. Die Verwendung einer Verbindung gemäß Anspruch 4 zur Herstellung eines Medikaments zur Verwendung bei der Behandlung von IBS (Reizkolon) und IBD (entzündlicher Darmerkrankung).

7. Eine Verbindung gemäß einer von Anspruch 1 bis 2 zur Behandlung von durch CRF (Corticoliberin) vermittelten Zuständen.

8. Ein Arzneimittel, umfassend eine Verbindung gemäß einer von Anspruch 1 bis 2 in Beimischung mit einem oder mehreren physiologisch verträglichen Trägem oder Exzipienten.

## Revendications

1. Composés de formule (Ia), y compris leurs stéréoisomères et leurs sels ou produits de solvatation pharmaceutiquement acceptables 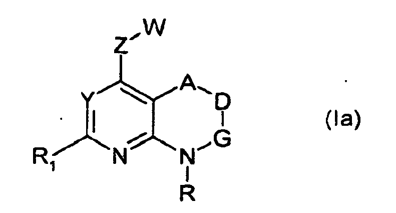 formule dans laquelle
R représente un groupe choisi entre des groupes : 2,4-dichloro- phényle, 2-chloro-4-méthylphényle, 2-chloro-4-trifluoro- méthylphényle, 2-chloro-4-méthoxyphényle, 2,4,5-triméthyl- phényle, 2,4-diméthylphényle, 2-méthyl-4-méthoxyphényle, 2-méthyl-4-éthoxyphényle, 2-méthyl-4-isopropoxyphényle, 2-méthyl-4-hydroxyphényle, 2-méthyl-4-chlorophényle, 2-méthyl-4-trifluorométhylphényle, 2,4-diméthoxyphényle, 2-méthoxy-4-trifluorométhylphényle, 2-méthoxy-4-chloro- phényle, 3-méthoxy-4-chlorophényle, 2,5-diméthoxy-4- chlorophényle, 2-méthoxy-4-isopropylphényle, 2-méthoxy- 4-trifluorométhylphényle, 2-méthoxy-4-isopropylphényle, 2-méthoxy-4-méthylphényle, 2-trifluorométhyl-4-chloro- phényle, 2,4-bis-trifluorométhylphényle, 2-trifluoro- méthyl-4-méthylphényle, 2-trifluorométhyl-4-méthoxy- phényle, 2-difluorométhyl-4-méthoxyphényle, 2-bromo-4- isopropylphényle, 2-méthyl-4-cyanophényle, 2-chloro-4- cyanophényle, 2-trifluorométhyl-4-cyanophényle, 2-trifluoro- méthoxy-4-cyanophényle, 2-éthyl-4-cyanophényle, 2-méthyl- 4-trifluorométhoxyphényle, 4-méthyl-6-diméthyl-amino- pyridine-3-yle, 2,6-bisméthoxy-pridine-3-yle, 2-méthyl- 6-méthoxy-pyridine-3-yle, 2-trifluorométhyl-6-méthoxy- pyridine-3-yl, 3-chloro-5-trichlorométhyl-pyridine-2-yle, 2-méthyl-4-(pyrazole-1-yl)-phényle, 2-méthoxy-4-(pyrazole- 1-yl)-phényle, 2,4,6-triméthoxyphényle, 2-méthyl-4,5- benzodioxolyle, 2-méthyl-3,4-benzodioxolyle ;
R₁ représente un groupe -CH₃ ;
R₃ représente un atome d'hydrogène ou un groupe alkyle en C₁ à C₆ ;
R₄ représente un atome d'hydrogène ou un groupe alkyle en C₁ à C₆ ;
R₅ représente un groupe alkyle en C₁ à C₆, halogénalkyle en C₁ à C₆, alkoxy en C₁ à C₆, halogénalkoxy en C₁ à C₆, cycloalkyle en C₃ à C₇, hydroxy, halogéno, nitro, cyano, -NR₃R₄ ;
R₇ représente un atome d'hydrogène ;
R₈ représente un atome d'hydrogène ;
R₉ représente un atome d'hydrogène ;
R₁₀ représente un atome d'hydrogène ;
R₁₁ représente un atome d'hydrogène ;
R₁₂ représente un atome d'hydrogène ;
R₁₃ représente un atome d'hydrogène ;
R₁₄ représente un groupe R₃ ;
D représente un groupe -CR₈R₉ ;
G représente un groupe -CR₁₀R₁₁ ou représente un groupe CR₁₀ ;
A représente un groupe CR₁₂R₁₃ ;
Y représente un atome d'azote ou un groupe -CRF ;
W représente un groupe 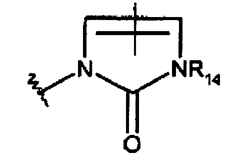
Z représente un groupe pyrazole qui peut être substitué avec 1 ou 2 groupes R₅.

2. Composés suivant la revendication 1, choisis dans le groupe suivant :
1-{1-[8-(2,4-dichlorophényl)-2-méthyl-5,6,7,8-tétrahydropyrido[2,3-d]pyrimidine-4-yl]-1H-pyrazole-3-yl}-2-imidazolidinone ;
1-{1-[8-(2,4-dichlorophényl)-2-méthyl-5,6,7,8-tétrahydro-1,8-nathtyridine-4-yl]-1H-pyrazole-3-yl}-2-imidazolidinone.

3. Procédé pour la préparation de composés de formule (Ia), comprenant les étapes suivantes : 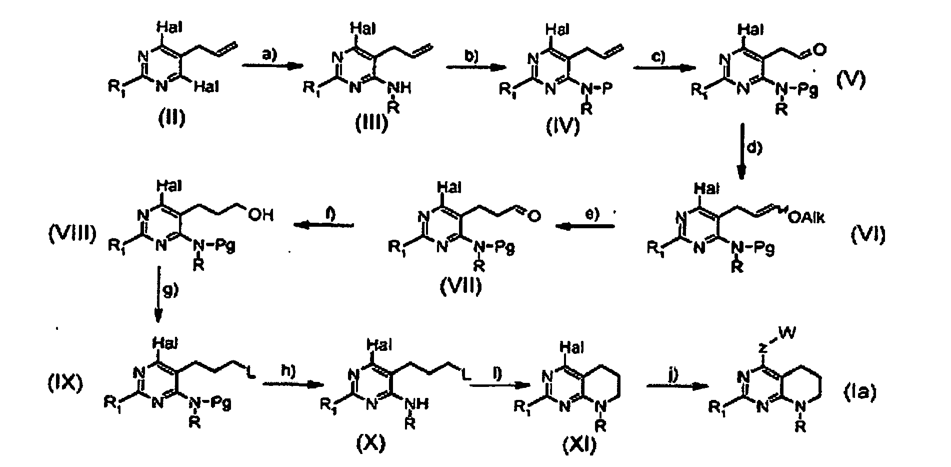 dans lesquelles :
l'étape a représente la substitution nucléophile avec une amine convenable (telle qu'une aniline substituée) de composés de formule (II), dans des conditions basiques (par exemple hydrure de sodium dans un solvant aprotique polaire), pour donner des composés (III) ;
l'étape b représente la protection du groupe amino avec un groupe protecteur convenable (tel qu'un groupe BOC) ;
l'étape c représente l'oxydation de la double liaison avec un agent oxydant convenable (tel que l'ozone dans un solvant protique polaire), pour l'aldéhyde des composés (V) ;
l'étape d + e représente la formation du groupe aldéhyde des composés (VII) par formation de l'éther d'énol par une réaction de Wittig dans les conditions habituelles, avec ensuite une hydrolyse acide (étape e) ;
l'étape f représente la réduction du groupe aldéhyde des composés (VII) en l'alcool des composés (VIII) avec un agent réducteur convenable (tel que le borohydrure de sodium) ;
l'étape g représente la conversion de l'alcool des composés (VIII) en un groupe partant convenable (tel que, par exemple, un groupe halogéno ou un résidu réactif d'un acide sulfonique (par exemple mésylate, tosylate), de préférence mésylate) ;
l'étape h représente la suppression de protection du groupe amino des composés (IX) ;
l'étape i représente la cyclisation intramoléculaire donnant les composés cyclisés (X) ;
l'étape j représente la conversion du dérivé halogéné, de préférence du chlorure, en les composés (Ia), par réaction avec le dérivé -Z-W réactif convenable, dans des conditions basiques (par exemple l'hydrure de sodium dans un solvant polaire).

4. Utilisation d'un composé suivant l'une quelconque des revendications 1 et 2, dans la préparation d'un médicament destiné à être utilisé dans le traitement d'affections à médiation par le CRF (facteur de libération de corticotrophine).

5. Utilisation d'un composé suivant la revendication 4, dans la préparation d'un médicament destiné à être utilisé dans le traitement de la dépression et de l'anxiété.

6. Utilisation d'un composé suivant la revendication 4, dans la préparation d'un médicament destiné à être utilisé dans le traitement du IBS (syndrome du côlon irritable) et de la IBD (maladie intestinale inflammatoire).

7. Composé suivant l'une quelconque des revendications 1 et 2, destiné au traitement d'affections à médiation par le CRF (facteur de libération de corticotrophine).

8. Composition pharmaceutique comprenant un composé de l'une quelconque des revendications 1 et 2, en mélange avec un ou plusieurs supports ou excipients physiologiquement acceptables.
